# EUROPEAN PATENT APPLICATION

(11) **EP 4 235 675 A1**
(43) Date of publication of application: **30.08.2023**
(21) Application number: 22159121.7
(22) Date of filing: 28.02.2022
(51) Int. Cl.: G16B 20/30, G16B 40/20, G16H 50/20

(54) **A METHOD FOR PREDICTING HLA B-CELL EPITOPES CONSIDERING ALLELE-SPECIFIC SURFACE ACCESSIBLE RESIDUES**

(71) Applicant: PIRCHE AG, 10627 Berlin (DE)
(72) Inventor: Niemann, Matthias, 10627 Berlin (DE)
(74) Representative: Hertin und Partner Rechts- und Patentanwälte PartG mbB

(57) **Abstract**

The invention relates to a computer-implemented method, system and/or computer product for predicting a human leukocyte antigen (HLA) B-cell epitope that is mismatched between two or more subjects. The invention relates to a computer-implemented method, system and/or computer product for producing a database of predicted solvent accessible surface residues for multiple human leukocyte antigen (HLA) proteins. According to the invention, the solvent accessible surface residues of the database are predicted by a neural network trained on amino acids sequences of experimentally determined and predicted HLA protein structures, and on the corresponding residues' surface accessibility.

## Description

The field of the invention relates to computer-implemented approaches for assessing immune reactions in the context of transplantation medicine.

In one aspect the invention relates to a computer-implemented method, system and/or computer product for predicting a human leukocyte antigen (HLA) B-cell epitope that is mismatched between two or more subjects.

In a further aspect the invention relates to a computer-implemented method, system and/or computer product for producing a database of predicted solvent accessible surface residues for multiple human leukocyte antigen (HLA) proteins.

The methods, systems and computer products described herein allow the identification of permissible HLA mismatches and therefore the provision of relatively safe transplantation material via computer-implemented analysis of HLA mismatches between e.g. a transplantation donor and a patient (recipient).

In embodiments, the invention relates to computer-implemented means that simulate biological phenomena in a novel approach towards assessing mismatched HLA B-cell epitopes and their predicted role in immune reactions in the context of transplantation medicine.

In one aspect the invention relates to a computer-implemented method for producing a database of predicted solvent accessible surface residues for multiple human leukocyte antigen (HLA) proteins, comprising a. Providing one or more HLA protein structures, for which a structure has been experimentally determined, b. Predicting HLA protein structure of one or more HLA proteins, for which a structure has not been experimentally determined, c. Supplementing the predicted HLA protein structures of b. with an HLA-bound peptide, if the structure of b. does not exhibit an HLA-bound peptide, d. Determining solvent accessible surface residues of each HLA protein structure of a. and c., and e. Generating a database of solvent accessible surface residues for the HLA proteins of a. and c. and HLA proteins additional to those of a. to c., comprising employing a neural network trained on amino acids sequences of the HLA proteins of a. and c. and the corresponding solvent accessible surface residues determined in d.

In one aspect the invention relates to a computer-implemented method for predicting a human leukocyte antigen (HLA) B-cell epitope that is mismatched between two or more subjects, comprising: i. Determining one or more mismatched amino acids of an HLA protein between two or more subjects, comprising preferably identifying amino acid similarity of an HLA protein of one subject with the same amino acid location in one or more HLA proteins of another subject, ii. Determining whether said mismatched amino acids are predicted to be positioned on a solvent accessible surface of an HLA structure, comprising comparing said mismatched amino acids to a database of solvent accessible surface residues for HLA proteins, wherein the solvent accessible surface residues of the database are predicted by a neural network trained on amino acids sequences of experimentally determined and predicted HLA protein structures, and on the corresponding residues' surface accessibility, iii. wherein a mismatched amino acid predicted to be surface accessible indicates a human leukocyte antigen (HLA) B-cell epitope that is mismatched between two or more subjects.

### BACKGROUND OF THE INVENTION

Transplantation of allogeneic material, such as cells, tissues and organs, is an evolving therapy that has become an increasingly attractive therapeutic option. The number of patients receiving transplants from unrelated donors is expected to continually increase in light of developments in assessing the risks of transplantation, and developments in effective therapeutic options in the context of transplantation medicine. Alloreactivity after transplantation has a major impact on clinical outcome, with pathological as well as beneficial effects. HLA mismatches are known to induce an immune reaction after transplantation, however the factors involved in predicting the risk of unwanted immune reaction are not completely understood.

Hematopoietic Stem Cell Transplantation (HSCT) is one example of a quickly growing therapeutic approach. The major limiting factor of HSCT remains the risk of graft-versus-host disease (GVHD), and since the number of patients receiving HSCT is expected to increase, the provision of novel approaches to prevent GVHD must be accelerated. To overcome the risk of GVHD, patients are preferably transplanted with a donor that is completely matched for all HLA-alleles. However, due to heterogeneity of HLA molecules in the population, these completely matched donors are not available for approximately 40% of patients. With increasing diversity of populations this issue become more pronounced, and it is expected to find optimal donors for even fewer patients. When a completely matched donor is not available, a clinician often has to face the difficult decision to choose the least harmful donor out of the mismatched donors (i.e. the one that carries the lowest GVHD risk). Similar problems face clinicians before selecting transplantation material for blood products, such as platelets, or cord blood or cord blood cell transplantations, kidney transplantations, or other transplantations at risk of side effects caused by unwanted alloreactivity.

Histocompatibility of patients and organ donors has been shown to correlate with long-term graft survival in organ transplantation. [1] Antibody recognition of donor antigen is dependent on the patient's immune reactivity towards determinants on donor-HLA, so-called antibody epitopes. The amino acid configuration and tertiary structure of these epitopes differs from the recipients' HLA proteins, allowing B cell receptors to distinguish self- and allo-protein, allowing for effective immune responses. [2] Several algorithms have been developed to predict B cell epitope mismatches between two individuals.

The HLAMatchmaker defines antibody-accessibility based on a few experimental crystal structures and groups amino acid positions and their corresponding configurations for defining so-called Eplets. [3] The EPRegistry database has been developed as a central repository listing these Eplets. [4,5] By defining residue-specific physicochemical properties, [6] provided potential explanations for epitope-specific antibody affinity.

The polymorphism of the HLA gene region and the pairing of two individuals in a transplantation setting however yields such a large number of combinations of HLA epitopes, that verifying each mismatched epitope indistinguishably by antibody reaction patterns is challenging, if possible at all. [7] It has been suggested that basically every amino acid mismatch is capable of triggering an immune response. [8,9] The HLA-EMMA algorithm further specifies this approach, by only considering polymorphic amino acid residues at solvent-accessible positions of HLA loci as potential B cell receptor targets. [10]

Given the large number of HLA alleles and the complexity of X-ray crystallography, it is not feasible to generate structural data for every HLA protein in order to identify surface residues. Thus, neural network predictors have been suggested to predict arbitrary proteins' surfaces. [11,12]

Despite the existing techniques, there exists a need for more effective and accurate methods for predicting whether donor material for a transplantation, which is HLA mismatched, is at increased risk of leading to a failed transplantation, for example development of GVHD, antibody or T cell mediated rejection (AMR/TCMR), and/or an increase in mortality.

### SUMMARY OF THE INVENTION

In light of the prior art the technical problem underlying the present invention is the provision of improved or alternative computer-implemented means for assessing histocompatibility between patients and organ donors. Another technical problem may also be considered as the provision of improved or alternative computer-implemented means for assessing mismatched HLA B-cell epitopes and their predicted role in immune reactions in the context of transplantation medicine These problems are solved by the features of the independent claims. Preferred embodiments of the present invention are provided by the dependent claims.

The invention therefore relates to computer-implemented means, such as methods, systems and computer products, for determining whether mismatched amino acids of an HLA molecule are predicted to be positioned on a solvent accessible surface of an HLA structure.

Embodiments of the invention comprise comparing mismatched amino acids to a database of solvent accessible surface residues for HLA proteins, wherein the solvent accessible surface residues of the database are predicted by a neural network trained on amino acid sequences of experimentally determined and predicted HLA protein structures, and on the corresponding residues' surface accessibility.

As described in greater detail below, the inventors have developed a deep learning prediction pipeline for solvent-accessibility defining allele-specific surface epitopes. The surface prediction methodology described in the examples, and in embodiments of the invention, is trained on experimental HLA structures of the Protein Data Bank (www.rscb.org), supplemented by HLA structures predicted by Deepmind's recent AlphaFold protein folding predictor. [13-15]

Due to the structural differences between HLA Class I and Class II proteins and the different distribution of available experimental structures, the prediction pipeline implementation of the present invention preferably applies Class- or locus-specific optimization. The results of the inventive HLA Class I-specific pipeline are shown in detail in the Examples below. Based on the inventive surface predictor, a matching algorithm can be implemented, for example as a web service, allowing for real-time application in transplantation diagnostics.

One aspect of the invention therefore relates to a computer-implemented method for producing a database of predicted solvent accessible surface residues for multiple human leukocyte antigen (HLA) proteins, wherein the solvent accessible surface residues of the database are predicted by a neural network trained on amino acid sequences of experimentally determined and predicted HLA protein structures, and on the corresponding residues' surface accessibility.

In one embodiment, the invention therefore relates to a computer-implemented method for producing a database of predicted solvent accessible surface residues for multiple human leukocyte antigen (HLA) proteins, comprising
a. Providing one or more HLA protein structures, for which a structure has been experimentally determined,
b. Predicting HLA protein structure of one or more HLA proteins, for which a structure has not been experimentally determined,
c. Supplementing the predicted HLA protein structures of b. with an HLA-bound peptide, if the structure of b. does not exhibit an HLA-bound peptide,
d. Determining solvent accessible surface residues of each HLA protein structure of a. and c., and
e. Generating a database of solvent accessible surface residues for the HLA proteins of a. and c. and HLA proteins additional to those of a. to c., comprising employing a neural network trained on amino acids sequences of the HLA proteins of a. and c. and the corresponding solvent accessible surface residues determined in d.

The generation of the database as described herein represents a novel approach towards predicting solvent accessible surface residues for human leukocyte antigen (HLA) proteins. By identifying such solvent accessible surface residues, potential B cell epitopes can be determined, thus indicating whether any given portion of an HLA sequence is presented on the surface of the intact molecule (solvent accessible) and may therefore represent a target for antibody production.

By generating a database for multiple, preferably essentially all, HLA protein structures, the likelihood of whether any given mismatched HLA amino acid (mismatched for example between a donor and recipient of a transplantation) is predicted to be surface (solvent) accessible can be assessed. For example, once the database is established, and a potential HLA mismatch is determined between a donor and recipient, that mismatched amino acid can be compared to the database to determine whether it is predicted to be surface accessible in the context of the HLA structure. If surface accessible, the risk of the mismatch representing a B cell epitope and thus potentially an antibody target can be determined.

To the knowledge of the inventors, employing a neural network trained only on (a) amino acid sequences of HLA proteins for which a structure has been experimentally determined, (b) HLA proteins for which a structure has not been experimentally determined, and (c) the solvent accessible surface residues of each HLA protein structure, a novel approach towards predicting solvent accessible surface residues of HLA proteins has been developed.

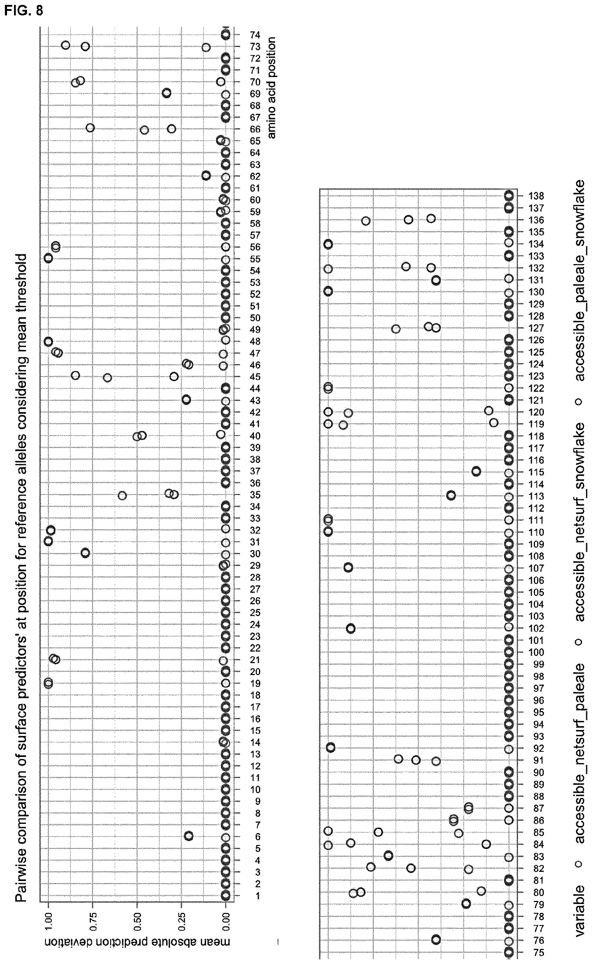

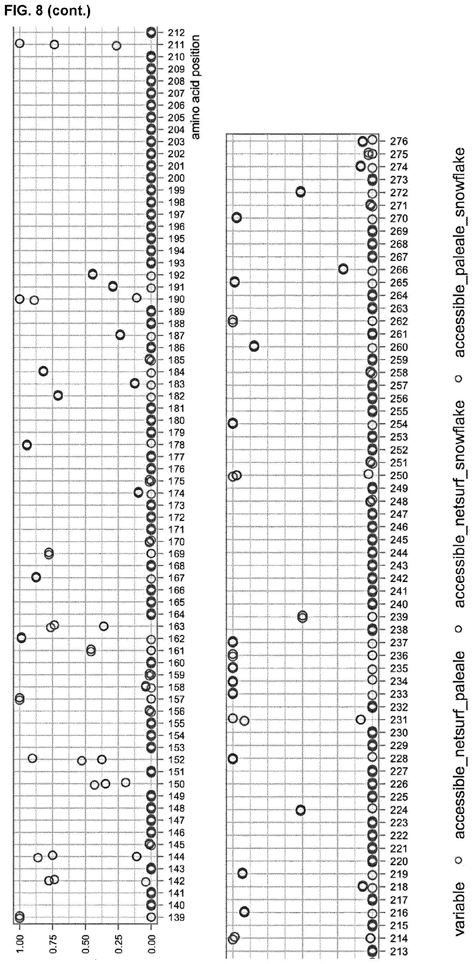

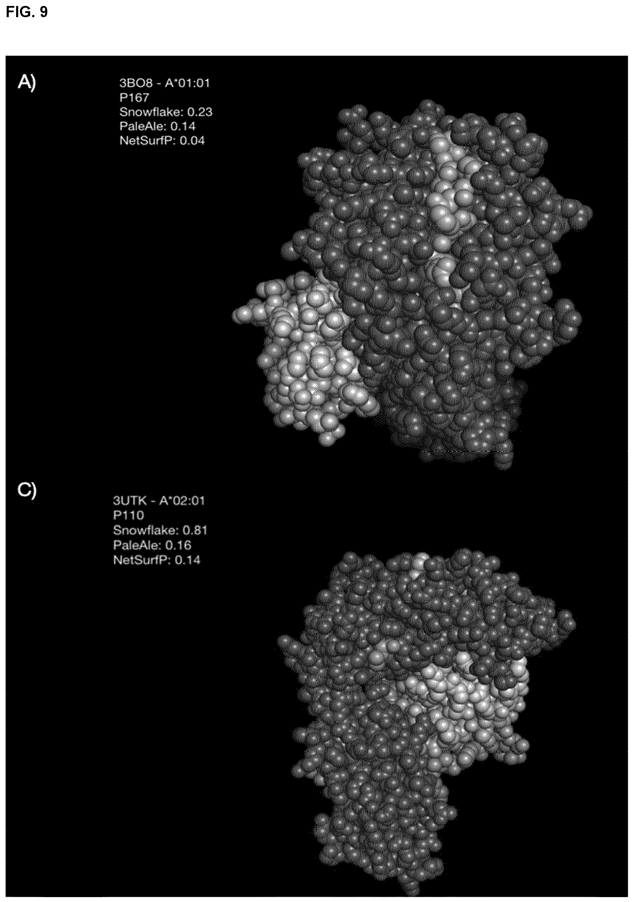

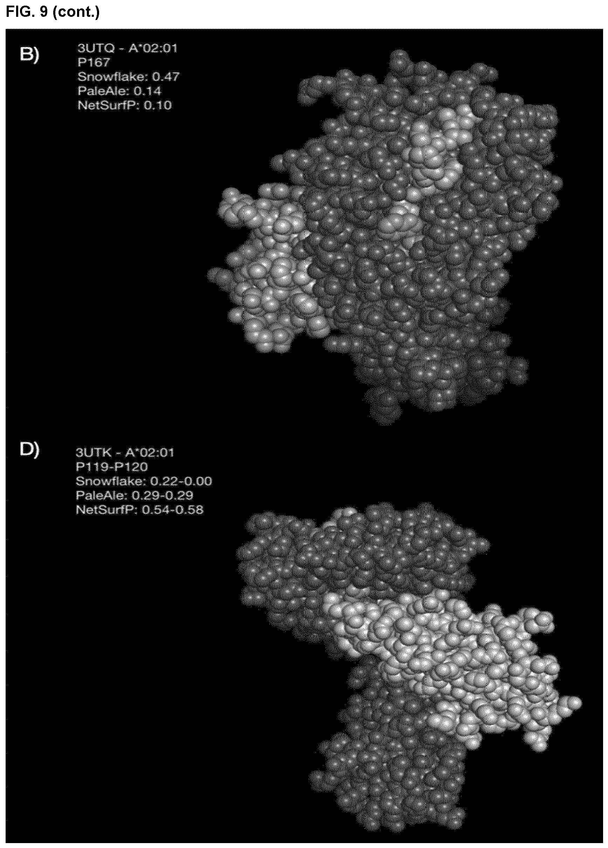

Despite some overlap in Eplets and B cell epitopes identified by the method of the present invention, it appears that the present invention leads to a prediction of distinct surface accessible residues, and in some cases appears to be more accurate than earlier approaches to determining B cell epitopes. For example, the recently described HLA-EMMA algorithm considers solvent-accessible amino acid mismatches as potential B cell receptor targets by defining solvent-accessible amino acid positions per HLA locus. An amino acid position is considered solvent-accessible, if PaleAle- or NetSurfP-solvent-accessibility scores exceed a certain threshold in at least one of the considered alleles of a locus. [10] This approach may however consider mismatched amino acids of two alleles as an epitope mismatch, although the amino acids within the respective alleles aren't surface-accessible (refer to the Examples below, and Figs 8, 9A and 9B), as the position's accessibility was determined by a different allele. Furthermore, HLA-EMMA considers amino acid positions as accessible, if at least one allele of the respective locus is predicted accessible by at least one of the considered surface prediction tools, which may increase the number of considered amino acid positions, once more experimental HLA structures are available. However, these new structures bias all alleles of the same HLA locus. Consequently, the present invention's approach towards determining allele-specific solvent-accessibility of mismatched amino-acids may increase specificity over earlier methods.

In the attached Examples, it has been shown that defined Eplets have spatial variance across different HLA-allelic proteins, despite having the same amino acid configuration. This may lead to defined Eplets being inaccessible although mismatched. Consequently, the predictive value of Eplets is hampered. The present invention however takes care of these configurations by defining an allele-specific epitope signature through a well-defined process.

The currently defined Eplets furthermore are inherently including HLA allele frequency data, biasing the Eplet definition towards infrequent alleles. However, it has been shown in the Examples, that exposed amino acid positions may be mismatched in rare recipient-allele combinations, that were not considered cases when defining Eplets. Consequently, Eplet matching (i.e. HLA Matchmaker) may underestimate epitope load in these cases. Due to its well-defined, consistent process of defining the allele-specific epitope signature, the present invention is better capable of predicting the corresponding epitope load.

To incorporate simulated HLA structures into surface prediction, the methods of the present invention implement a novel HLA surface predictor comparable to both NetSurfP 2.0 and PaleAle 4.0. These predictors implement a deep learning neural network architecture using BRNN to predict (amongst other characteristics) relative surface accessibility. Both networks have been trained with the experimental structures reported in the PDB. [11,12] Limiting on solvent accessibility as predicted output, the present invention's deep learning model is preferably trained exclusively on HLA experimental structures, and complemented by predicted HLA structures.

Although HLA Class I experimental structures showed little overall structural variance in dihedral angles between alleles (Examples, Fig 5), solvent accessibility varied across different alleles, indicating solvent accessibility to be allele-specific (Examples, Fig 6). Visual representations of characteristic positions support this hypothesis (Examples, Fig 9).

Consequently, amino acid matching approaches may be improved using the present invention by considering solvent accessibility of the respective amino acid of the specific allele, providing also a consistent explanation for epitopes' directionality.

Previous B cell epitope matching approaches considered HLA protein structures of different alleles of the same locus or Class being conserved despite the alleles' heterogeneity. The present invention unexpectedly found allele-specific differences in the solvent accessibility of amino acid residues that suggest considering the surface on an individual basis rather than assuming identical structure.

In one embodiment, the HLA protein structure of a. to c. and the solvent accessible surface residues of d. comprise:
(i) an extracellular alpha chain, a beta-2-microglobulin and an HLA-bound peptide structure, in case of HLA Class I proteins, or
(ii) the protein's extracellular alpha and beta chains, and an HLA-bound peptide structure, in case of HLA Class II proteins.

The consideration of these complete HLA structures, including e.g. alpha and beta chains, with the HLA-bound peptide, appears to represent an improvement over earlier approaches, and enables a more accurate determination of which residues of the HLA protein sequence are in fact solvent (surface) accessible and thus a potential B cell epitope target.

In one embodiment, the supplementing according to step c. comprises:
a. For predicted structures with known binding peptides, adding said known peptides to the HLA protein structure, and/or
b. For predicted structures without known binding peptides, adding peptides to the HLA protein structure that are known to bind to an HLA allele with a high degree (the greatest degree) of amino acid sequence identity in extracellular domains to the HLA amino acid sequence of said predicted structure without known binding peptides.

This approach therefore is carried out in consideration of HLA structures bound by peptide sequences known to bind the HLA protein (allele-specific), or peptides known to bind similar HLA proteins (based on HLA sequence similarity), thus an accurate combined representation of HLA structure, including both the HLA molecule itself and any peptide presented by said HLA, is assessed for potential surface resides.

In some embodiments, determining solvent accessible surface residues of an HLA protein structure according to step d. comprises employing a rolling-probe algorithm, preferably a Shrake-Rupley rolling-probe algorithm, a Half Sphere Exposure (HSE) algorithm, a linear approximation algorithm or a power diagram-based algorithm.

The above-mentioned algorithms for determining solvent accessible surface residues are functional and, preferably in combination with the peptide-bound HLA structures, appear to lead to improved prediction of solvent accessibility. Additional means and algorithms for determining solvent accessible surface residues are known to a skilled person and may be employed accordingly.

In some embodiments, the neural network according to step e. comprises a long short-term memory bidirectional recurrent neural network.

In some embodiments, the neural network according to step e. comprises the HLA alpha chain (for HLA Class I) or the HLA alpha and beta chains (for HLA Class II) amino acid sequence(s) as an input and the residue-wise solvent accessible surface as an output.

This constellation of neural network type, and in- and output information, leads to a beneficial training set with which surface accessibility may be determined with greater accuracy than earlier approaches.

In some embodiments, the database according to step e. comprises solvent accessible surface residues for essentially all HLA proteins (all HLA alleles) in any given HLA Class.

By generating such a database, an optimal reference data set is provided to which any given HLA mismatch can be compared, for example prior to transplantation, in order to determine a risk of a B cell/antibody-mediated immune reaction against said mismatch. It must be considered that any given learning-based surface prediction is obsolete if predicted structures have been generated for all known HLA alleles. However, structure prediction is time and computer-power costly, thus making it practically challenging, if not entirely implausible, to have to predict structural information for all HLA alleles. In order to present a complete or near complete database, the present invention, in embodiments, employs a subset of determined HLA structures, combined with a subset of *in silico* predicted HLA structures, and a prediction of accessible binding residues, to train a neural network capable of assessing other HLA alleles/sequences. Thus, the neural network of step e. is trained on this combination of data sets, and subsequently allows a machine learning process to determine the solvent accessible residues for any number of additional HLA alleles based on their protein sequence.

In embodiments, the neural network according to e. is HLA Class and/or HLA-locus specific, for example comprises multiple solvent accessible surface residues for multiple human leukocyte antigen (HLA) proteins from only HLA Class I proteins, only HLA Class II proteins, or only for alleles selected from the same HLA locus.

Thus, a database can be generated as described herein, in which essentially all known HLA alleles of a given Class or HLA locus are assessed, and upon determining e.g. a mismatch between donor and recipient of transplantation material, the relevant database can be considered. This method enables a more efficient and/or quicker approach to interrogating relevant HLA mismatches, depending on the HLA Class of locus of interest.

In embodiments, the method is automated, preferably wherein the method does not comprise manual curation of the database according to verified antibody binding.

In contrast to other B cell epitope predictors known in the art, the present invention preferably does not rely on manual curation of the database based on known HLA epitopes, for example known epitopes to which antibodies have been detected. The absence of manual curation represents an improvement in automation in this approach, and indicates that the means of the present invention are equally effective, if not more accurate, in predicting surface accessible residues, compared to older approaches which require or employ manual curation of the database based on known epitopes bound by antibodies.

The well-defined process of the present invention allows more reliable definition of epitopes compared to manually annotated concepts, as it also covers consistent epitope prediction for uncommon patient-donor constellations. Given an algorithm's consistency is a crucial requirement by regulatory bodies, the present invention has a clear advantage over previous concepts.

The invention therefore further relates, in related aspects, to a computer system for producing a database of predicted solvent accessible surface residues for multiple human leukocyte antigen (HLA) proteins, comprising one more computer hardware components, such as a computer processor or data storage device, and optionally other hardware components, configured for:
a. Providing one or more HLA protein structures, for which a structure has been experimentally determined,
b. Predicting HLA protein structure of one or more HLA proteins, for which a structure has not been experimentally determined,
c. Supplementing the predicted HLA protein structures of b. with an HLA-bound peptide, if the structure of b. does not exhibit an HLA-bound peptide,
d. Determining solvent accessible surface residues of each HLA protein structure of a. and c., and
e. Generating a database of solvent accessible surface residues for the HLA proteins of a. and c. and HLA proteins additional to those of a. to c., comprising employing a neural network trained on amino acids sequences of the HLA proteins of a. and c. and the corresponding solvent accessible surface residues determined in d.

The invention therefore further relates, in related aspects, to a computer product, such as a software, or kit comprising computer software, or to an online portal offering a software-based service, for producing a database of predicted solvent accessible surface residues for multiple human leukocyte antigen (HLA) proteins, comprising
a. Providing one or more HLA protein structures, for which a structure has been experimentally determined,
b. Predicting HLA protein structure of one or more HLA proteins, for which a structure has not been experimentally determined,
c. Supplementing the predicted HLA protein structures of b. with an HLA-bound peptide, if the structure of b. does not exhibit an HLA-bound peptide,
d. Determining solvent accessible surface residues of each HLA protein structure of a. and c., and
e. Generating a database of solvent accessible surface residues for the HLA proteins of a. and c. and HLA proteins additional to those of a. to c., comprising employing a neural network trained on amino acids sequences of the HLA proteins of a. and c. and the corresponding solvent accessible surface residues determined in d.

The invention further relates to a database and/or data structure, generated using the method, system and/or computer product described herein.

Any embodiment of the invention disclosed in the context of the method, system or computer product, is considered disclosed in combination with the other related aspects of the invention. For example, the features of the method apply to the system, and vice versa.

A further aspect of the invention relates to a computer-implemented method for predicting a human leukocyte antigen (HLA) B-cell epitope that is mismatched between two or more subjects, comprising comparing a mismatched amino acid to a database of solvent accessible surface residues for HLA proteins, wherein the solvent accessible surface residues of the database are predicted by a neural network trained on amino acid sequences of experimentally determined and predicted HLA protein structures, and on the corresponding residues' surface accessibility.

In one embodiment, the invention therefore relates to a computer-implemented method for predicting a human leukocyte antigen (HLA) B-cell epitope that is mismatched between two or more subjects, comprising
i. Determining one or more mismatched amino acids of an HLA protein between two or more subjects,
   - comprising preferably identifying amino acid similarity of an HLA protein of one subject with the same amino acid positions in one or more HLA proteins of another subject,
ii. Determining whether said mismatched amino acids are predicted to be positioned on a solvent accessible surface of an HLA structure,
   - comprising comparing said mismatched amino acids to a database of solvent accessible surface residues for HLA proteins, wherein the solvent accessible surface residues of the database are predicted by a neural network trained on amino acids sequences of experimentally determined and predicted HLA protein structures, and on the corresponding residues' surface accessibility,
iii. wherein a mismatched amino acid predicted to be surface accessible indicates a human leukocyte antigen (HLA) B-cell epitope that is mismatched between two or more subjects.

Through this method, one can ascertain whether any given mismatched HLA amino acid, for example between a donor and recipient of transplantation material, is predicted to be surface (solvent) accessible and therefore a potential B cell epitope. In embodiments, this aspect of the invention is also defined by the solvent accessible surface residues of the database being predicted by a neural network trained on amino acid sequences of experimentally determined and predicted HLA protein structures, and on the corresponding residues' surface accessibility.

The advantages of the database of the invention, as described above, are also applicable to the method of the invention for predicting a human leukocyte antigen (HLA) B-cell epitope that is mismatched between two or more subjects.

In embodiments, the computer-implemented method for predicting an HLA B-cell epitope that is mismatched between two or more subjects produces a score. The score reflects the number of allele-specific surface-accessible amino acid mismatches identified between two subjects. As demonstrated in the Examples below, clinical data demonstrates that the number of allele-specific surface-accessible amino acid mismatches (i.e. Snowflake score, according to the Examples) was significantly associated with immunizer-specific antibody (ISA) development. Thus the method of the present invention provides not only an assessment of HLA B cell epitopes from mismatched HLA proteins, but provides some indication of the likelihood of immunizer-specific antibody (ISA) development, thus an assessment of immunogenicity may be inferred from the present method.

In one embodiment, the method of the invention for predicting a human leukocyte antigen (HLA) B-cell epitope that is mismatched between two or more subjects, is characterized in that the database is produced using a method for producing a database of predicted solvent accessible surface residues for multiple human leukocyte antigen (HLA) proteins, as described above.

In one embodiment, a mismatched amino acid predicted to be surface accessible indicates an antibody immune response against the corresponding mismatched HLA protein.

The method as described herein therefore also provides an indication of whether a mismatched HLA amino acid is immunogenic, and thus potentially a risk factor in transplantation of material between HLA mismatched subjects.

In a further aspect, the invention relates to a computer-implemented method for selecting transplantation material for allogeneic transplantation between subjects with one or more human leukocyte antigen (HLA) mismatches, comprising assessing whether one or more human leukocyte antigen (HLA) B-cell epitopes are mismatched between said subjects according to the method as described herein, wherein the number of determined mismatched HLA B-cell epitopes positively correlates with an antibody immune response against a corresponding mismatched HLA protein and/or a likelihood of an adverse immune reaction after transplantation.

In embodiments of the invention, the transplantation comprises solid organ transplantation (such as kidney transplantation, liver transplantation, heart transplantation, lung transplantation), transplantation of stem cells (such as transplantation of hematopoietic stem-cells (HSCT), cord blood or cord blood cells), or transfusion of blood or cell products (such as platelet transfusion).

In one embodiment, the method comprises additionally determining one or more mismatched HLA T-cell epitopes between said subjects, preferably comprising determining the number of predicted indirectly recognized HLA epitopes (PIRCHEs), wherein said PIRCHEs are recipient-or donor-specific HLA-derived peptides from a mismatched recipient or donor HLA allele, respectively, and are predicted to be presented by an HLA molecule, wherein the number of determined mismatched HLA T-cell epitopes positively correlates with a likelihood of an adverse immune reaction after transplantation.

Any embodiment of the invention disclosed in the context of the method, system or computer product, is considered disclosed in combination with the other related aspects of the invention. For example, the features of the method apply to the system, and vice versa. The embodiments or features of any given aspect may also be considered in combination with the features or embodiments of another aspect of the invention.

A further embodiment of the invention therefore relates to a system for selecting and/or screening donor material for transplantation, for example for selecting donor material with permissible mismatches from HLA mismatched donors, comprising one or more software modules capable of carrying out one or more methods as described herein.

In one embodiment the method as described herein is characterised in that the information stored in the database is an electronically-stored representation of biological data, or corresponds to, or is an abstraction of, one or more biological entities. The present invention therefore relates to a computer implemented method in which data are processed that correspond to "real-world" biological entities. The database as such is not only characterized by the means of its production, but preferably also by the particular biological context of the entities and relationships embodied in the database.

The novel database of the invention and its use in the methods described herein enables potentially improved (shortened) processing times in comparison to those approaches attempted previously.

In one embodiment, the method as described herein is characterised in that HLA typing and/or matching between one or more subjects, for example donor and recipient of transplantation material, has been conducted in advance of carrying out the method described herein. The HLA-typing data of the method may have been carried out in advance, and the data regarding HLA-type subsequently analysed via the methods of the present invention.

In one embodiment the HLA protein sequences relate preferably to an informational and/or computational representation of an HLA allele. In a preferred embodiment the HLA sequences relate to alleles that are defined by a unique protein sequence, whereby HLA alleles that show only DNA sequence differences without changes in the corresponding protein sequence are in some embodiments preferably not considered in the method. An example of such HLA alleles that show only DNA sequence differences relate to allele-specific features described in fields 3 and 4 of the standard HLA nomenclature system relating to synonymous DNA sequence changes or sequence changes outside a coding region.

In one embodiment the selected HLA molecules for assessment are the HLA loci A*, B*, C*, DRB1* and/or DQB1*, and optionally DQA1*, DPA1*, DPB1* and DRB3/4/5*.

A further aspect of the invention relates to a system for selecting and/or screening donor material for transplantation, for example for selecting donor material with permissible mismatches from mismatched unrelated donors, comprising software modules for determining B cell epitopes, as described herein. In one embodiment, the system for selecting and/or screening donor material for transplantation is characterised in that said HLA software module carries out HLA matching between donor and recipient HLA typing data on the basis of high-resolution allele coding (e.g. A*02:01), preferably for HLA loci A*, B*, C*, DRB1* and/or DQB1*, and optionally DQA1*, DPA1*, DPB1* and DRB3/4/5*.

In one embodiment, the system for selecting and/or screening donor material for transplantation is characterised in that said HLA software module carries out HLA epitope matching between donor and recipient HLA typing data on low or intermediate resolution data (i.e. ambiguous HLA typing). By multiple imputation of potential genotypes matching with the given low or intermediate resolution input data and weighting genotypes by haplotype frequencies, multiple epitope match results are generated for a single recipient donor pair. These results are aggregated together based on said haplotype frequencies. Consequently, the invention presented herein is capable of being used in settings, where high resolution HLA typing data is not available with current technologies, e.g. due to time constraints (e.g. deceased donor kidney allocation). Opposed to that, existing algorithms for B cell epitope matching require protein-level typing for both recipient and donor.

In some embodiments, the method comprises additionally incorporating the information generated by the methods of the present invention for multiple donors and/or multiple recipients, for example wherein the data outputted by the present invention is incorporated in a list of HLA mismatched donors and/or recipients with acceptably low or unacceptably high numbers of B cell epitopes, in the context of any given transplantation.

The various embodiments of the present invention provide approaches towards HLA matching providing a unique service to transplantation providers searching for transplantation material with low risk of causing unwanted allogenic reactions. The technical effect of the invention described herein is not only the improved speed of interrogation of donor databanks, but also the provision of safer transplantation material, thereby increasing the health of the patient population having received transplantations of allogenic material.

In one embodiment, donor profiles are preferably stored in an additional data structure, database, and/or library. In one embodiment, multiple donor profiles or donor types are stored in a donor data structure, database, and/or library comprising preferably essentially all possible donors for essentially all possible genotypes or possible combinations of HLA alleles, and/or haplotypes.

The phrase "essentially all" relates in this context to a significant number of, preferably all, or every known, protein variant of the allele group. The method therefore can effectively determine whether a particular donor or allele group indeed is associated with a relatively low number of predicted B cell epitopes and therefore present itself as a preferred selection.

Because those skilled in the art are able to make a pre-selection of possible protein variants within any given allele group, either on the basis of the reliability of the stored data or on population frequencies of certain alleles, the method is not limited to analysis of all known protein sequences of an allele group. In one embodiment the method will encompass the analysis of essentially all known (or relevant) protein sequences within the allele group, in order to provide more definitive results.

In one embodiment, the invention enables searching with recipient information (corresponding preferably to a biological recipient in need of a transplantation) against one or more donor profiles, wherein the result (outcome) of the present invention provides information on the one or more (preferably multiple) best-matched mismatched donor profiles, preferably a mismatched donor with a lowest possible number of determined (predicted) B cell epitopes.

In one embodiment the invention is characterized in that the method and/or system is functionally connected to electronic registers of donor or patient material, wherein the outcome of the method and/or system of the invention enables a query to be sent from an end-user of said method and/or system to said register for any given one or more best-matched mismatched donor material corresponding to said outcome. The invention preferably links the end user, for example a medical practitioner or transplant provider, with the stem cell, organ or other donor material registers that comprise information on said donor material.

The transplantation material may relate to any given biological matter intended for transplantation. Such biological matter may relate to cells, organs, tissue, blood, in particular cord blood, hematopoietic stem cells, or other progenitor or stem cell populations, or organs for transplantation. Preferred relevant transplantation fields are solid organ transplantation (such as kidney transplantation, liver transplantation, heart transplantation, lung transplantation), transplantation of stem cells (such as transplantation of hematopoietic stem-cells (HSCT), cord blood or cord blood cells), or transfusion of blood or cell products (such as platelet transfusion).

The computer implementation of the invention enables an efficient, fast and reliable method for identification of potentially permissible donor material for allogeneic transplantation. The data processed by the software can be handled in a completely or partially automatic manner, thereby enabling in a preferred embodiment an automated computer-implemented method. Data regarding the HLA typing of donor and recipient, in addition to the number of B cell epitopes determined for any donor-recipient pair, can be stored electronically and eventually maintained in appropriate databases. The invention therefore also relates to computer software capable of carrying out the method as described herein.

In one embodiment, the system of the invention preferably comprises one or more databases with information on all published HLA alleles. The database may be updated as new HLA allele sequences are published. Computer software, which could also be based on any given computing language, can be used for generating and/or updating the databases, in addition to calling the respective programmes required.

In embodiments, the invention further comprises a method and/or system for preferably pre-transplantation prediction of an immune response against human leukocyte antigens (HLA), which may occur after transplantation. The system may comprise computing devices, data storage devices and/or appropriate software, for example individual software modules, which interact with each other to carry out the method as described herein.

In one embodiment the present invention relates to a method as described herein, wherein HLA typing is obtained for and/or carried out for HLA subtypes HLA-A, HLA-B, HLA-C, HLA-DRB1 , HLA-DQB1 , HLA-DPB1 , -DQA1 , -DPA1 , -G, -E, -F, MICA, MICB and/or KIR. In one embodiment the present invention relates to a method as described herein, wherein HLA typing is provided and/or carried out on HLA-A, -B, -C, -DRB1 and -DQB1. In the domain of stem cell transplantation, these 5 alleles provide the basis for the commonly used terminology "9/10-matched", which refers to a 9/10-matched unrelated donor. In such a case 9 of the 10 alleles of these 5 genes show a match but one remaining allele does not match. The present invention therefore allows, in one embodiment, determination of whether the use of material from such a 9/10-matched unrelated donor is safe, i. e. whether the mismatch is permissible for transplantation. In the domain of solid organ transplantation, sometimes only a subset of the named HLA subtypes (e.g. HLA-A, HLA-B and HLA-DRB1) is focused on.

The invention relates also to the method as described herein for finding permissible HLA-mismatches in donor samples where more mismatches are present than the common 9/10 scenario. One example of potential donors who are encompassed by the present invention are Haploidentical donors, who may be screened using the method described herein for permissible mismatched donor material. A haploidentical related donor, may be described as a donor who has a "50% match" to the patient. This type of donor can be a parent, sibling, or child. By definition, a parent or child of a patient will always be a haploidentical donor since half of the genetic material comes from each parent. There is a 50% chance that a sibling will be a haploidentical donor. Haploidentical HSCT offers many more people the option of HCT as 90% of patients have a haploidentical family member. Other advantages include: immediate donor availability; equivalent access for all patients regardless of ethnic background; ability to select between multiple donors; and ability to obtain additional cells if needed. Alternatively, cord blood units (CBUs) may not show a high level of HLA-matching, but still be suitable for transplantation if the mismatches are permissible as determined by the method as described herein. CBU's are typically minimally 4/6 matched, but this match can however lead to a 4/10 or 5/10 situation at the allelic level.

In one embodiment the present invention relates to a method as described herein, wherein HLA typing comprises serological and/or molecular typing. In a preferred embodiment the present invention relates to a method as described herein, wherein HLA typing is carried out at high resolution level with amino acid sequence-based typing.

In one embodiment the present invention relates to a method as described herein, wherein HLA typing comprises sequencing of exon 1-7 for HLA class I alleles and exon 1-6 for HLA class II alleles. In one embodiment the present invention relates to a method as described herein, wherein HLA typing comprises high resolution HLA-A, -B, -C, -DRB1 and -DQB1 typing of exons 2 and 3 for HLA class-I alleles and exon 2 for HLA class-II alleles. These particular HLA-typing approaches are described in the examples provided herein and demonstrate advantages over earlier typing methods, providing full coverage of the alleles that need to be typed to identify the mismatches.

### DETAILED DESCRIPTION OF THE INVENTION

The invention therefore relates to computer-implemented means, such as methods, systems and computer products, for determining whether mismatched amino acids of an HLA molecule are predicted to be positioned on a solvent accessible surface of an HLA structure. Embodiments of the invention comprise comparing mismatched amino acids to a database of solvent accessible surface residues for HLA proteins, wherein the solvent accessible surface residues of the database are predicted by a neural network trained on amino acid sequences of experimentally determined and predicted HLA protein structures, and on the corresponding residues' surface accessibility.

As used herein, "solvent accessible surface residues" relates to any amino acid, for example of an HLA protein, that is determined by a computer-implemented method to be accessible to solvent, for example water, when the protein is in an aqueous environment. The term "surface accessible" may be used interchangeably. Other terms relate to the accessible surface area (ASA) or solvent-accessible surface area (SASA) of a protein, commonly understood as the surface area of a protein that is accessible to a solvent. Various methods for determining the solvent accessible surface residues of a biomolecule are available, for example ASA was first described by Lee & Richards in 1971 and is sometimes called the Lee-Richards molecular surface. ASA is often calculated using a 'rolling ball' algorithm, for example as developed by Shrake & Rupley in 1973, which uses a sphere (of solvent) of a particular radius to 'probe' the surface of the molecule.

By way of example, the Shrake-Rupley algorithm is a numerical method that draws a mesh of points equidistant from each atom of the molecule and uses the number of these points that are solvent accessible to determine the surface area. The points are drawn at a water molecule's estimated radius beyond the van der Waals radius, which is effectively similar to 'rolling a ball' along the surface. All points are checked against the surface of neighboring atoms to determine whether they are buried or accessible. The number of points accessible is multiplied by the portion of surface area each point represents to calculate the ASA. The choice of the 'probe radius' does have an effect on the observed surface area, as using a smaller probe radius detects more surface details and therefore reports a larger surface. A typical value is 1.4Å, which approximates the radius of a water molecule (Shrake, A; Rupley, JA. (1973). "Environment and exposure to solvent of protein atoms. Lysozyme and insulin". J Mol Biol. 79 (2): 351-71). Alternative methods are described for example in Ali et al (A Review of Methods Available to Estimate Solvent-Accessible Surface Areas of Soluble Proteins in the Folded and Unfolded States, 2014, Curr Protein Pept Sci. 2014;15(5):456-76).

As used herein, an "HLA protein structure" refers to a three-dimensional computer representation of the structure of an HLA protein. HLA protein structures can be obtained via various methods, including analysis of crystal structures.

Protein crystallization is the process of formation of a regular array of individual protein molecules stabilized by crystal contacts. If the crystal is sufficiently ordered, it will diffract. In the process of protein crystallization, proteins are dissolved in an aqueous environment and sample solution until they reach the supersaturated state. Different methods are used to reach that state such as vapor diffusion, microbatch, microdialysis, and free-interface diffusion. Once formed, these crystals can be used in structural biology to study the molecular structure of the protein, particularly for various industrial or medical purposes. Macromolecular structures can be determined from protein crystals using a variety of methods, including X-Ray Diffraction/X-ray crystallography, Cryogenic Electron Microscopy (CryoEM) (including Electron Crystallography and Microcrystal Electron Diffraction (MicroED)), Small-angle X-ray scattering, and Neutron diffraction. Computer representations of the structure can be generated using standard methods established in the art.

As used herein, the term "a structure that has been experimentally determined" relates to an HLA protein structure that has been experimentally assessed and a crystal structure has been determined and analysed.

As used herein, the term "a structure that has not been experimentally determined" relates to an HLA protein structure that has not been experimentally determined but has been predicted using a computer-implemented method of protein structure prediction. For example, a "protein structure of one or more HLA proteins, for which a structure has not been experimentally determined" may be used interchangeably with "a protein structure of one or more HLA proteins, predicted by a computer-implemented method".

Protein structure prediction is considered the inference of the three-dimensional structure of a protein from its amino acid sequence. Proteins are chains of amino acids joined together by peptide bonds. Many conformations of this chain are possible due to the rotation of the chain about each alpha-Carbon atom (Cα atom). It is these conformational changes that are responsible for differences in the three-dimensional structure of proteins.

Secondary structure prediction is a set of techniques in bioinformatics that aim to predict the local secondary structures of proteins based on knowledge of amino acid sequence. For proteins, a prediction consists of assigning regions of the amino acid sequence as likely alpha helices, beta strands or turns. The success of a prediction can be determined by comparing it to the results of the DSSP algorithm (or similar e.g. STRIDE) applied to the crystal structure of the protein. Specialized algorithms have been developed for the detection of specific well-defined patterns such as transmembrane helices and coiled coils in proteins. Multiple methods are available to predict structure and a high accuracy can be obtained using e.g. machine learning and sequence alignments. The high accuracy allows the use of the predictions to improve fold recognition and ab initio protein structure prediction, classification of structural motifs, and refinement of sequence alignments.

Tertiary structure prediction provides a predicted structure of the three-dimensional shape of a protein. The tertiary structure will have a single polypeptide chain "backbone" with one or more protein secondary structures, the protein domains. Amino acid side chains may interact and bond in several ways. The interactions and bonds of side chains within a particular protein determine its tertiary structure. Tertiary structure prediction methods typically involve "comparative" or "homology modelling" and "fold recognition" methods. Alternatively, "de novo protein structure prediction" involves an algorithmic process by which protein tertiary structure is predicted from its amino acid primary sequence. Various methods for predicting protein structure are available, such as are reviewed in Pearce et al (JBC, Volume 297, Issue 1, 2021) or Susanty et al (BIO Web of Conferences 41, 04003, 2021).

If a protein of known tertiary structure shares at least 30% of its sequence with a potential homolog of undetermined structure, comparative methods that overlay the putative unknown structure with the known can be utilized to predict the likely structure of the unknown. However, below this threshold three other classes of strategy are used to determine possible structure from an initial model: ab initio protein prediction, fold recognition, and threading. Ab Initio Methods: In ab initio methods, an initial effort to elucidate secondary structures (alpha helix, beta sheet, beta turn, etc.) from primary structure is made by utilization of physicochemical parameters and neural net algorithms. From that point, algorithms predict tertiary folding. One drawback to this strategy is that it is not yet capable of incorporating the locations and orientation of amino acid side chains. Fold Prediction: In fold recognition strategies, a prediction of secondary structure is first made and then compared to either a library of known protein folds, such as CATH or SCOP, or what is known as a "periodic table" of possible secondary structure forms. A confidence score is then assigned to likely matches. Threading: In threading strategies, the fold recognition technique is expanded further. In this process, empirically based energy functions for the interaction of residue pairs are used to place the unknown protein onto a putative backbone as a best fit, accommodating gaps where appropriate. The best interactions are then accentuated in order to discriminate amongst potential decoys and to predict the most likely conformation. The goal of both fold and threading strategies is to ascertain whether a fold in an unknown protein is similar to a domain in a known one deposited in a database, such as the protein databank (PDB). This is in contrast to de novo (ab initio) methods where structure is determined using a physics-base approach en lieu of comparing folds in the protein to structures in a data base.

In one embodiment, the predicted HLA protein structure is provided using an ab initio protein structure prediction. In one embodiment, the predicted HLA protein structure is provided using a fold recognition prediction. In one embodiment, the predicted HLA protein structure is provided using a threading prediction.

Examples of protein structure prediction that may be employed are known in the art, and include techniques described by Jumper et al (Highly accurate protein structure prediction with AlphaFold. Nature 596, 583-589, 2021) or Baek et al (Accurate prediction of protein structures and interactions using a three-track neural network, Science, 19 Aug 2021, Vol 373, Issue 6557, pp. 871-876).

As used herein, the term "supplementing the predicted HLA protein structures with an HLA-bound peptide" refers to adding a bound peptide to the predicted structure of an HLA protein, such as a structure of an HLA alpha chain and a Beta-2-microglobulin or HLA alpha and beta chain.

As used herein, the term "neural network" refers to a neural network as understood by a skilled person. For example, a neural network is a network or circuit of neurons in an artificial neural network, composed of artificial neurons or nodes, connected by edges. Artificial neural networks (ANNs), also named neural networks (NNs), are based on a collection of connected units or nodes called artificial neurons, which loosely model the neurons in a biological brain.

An artificial neural network preferably consists of a collection of simulated neurons. By way of example, each neuron is a node which is connected to other nodes via links that correspond loosely to biological axon-synapse-dendrite connections. Each link has a weight, which determines the strength of one node's influence on another. The neurons are typically organized into multiple layers, especially in deep learning. Neurons of one layer connect only to neurons of the immediately preceding and immediately following layers. The layer that receives external data is the input layer. The layer that produces the ultimate result is the output layer. In between them may be zero or more hidden layers. Single layer and unlayered networks may also be used. Example neural networks are known to a skilled person and are described in more detail in the Examples below.

As used herein, "training" a neural network refers to the term as commonly understood by a skilled person. By way of example, neural networks learn (or are trained) by processing examples, each of which contains a known "input" and "output," forming probability-weighted associations between the two, which are stored within the data structure of the net itself. The training of a neural network from a given example is usually conducted by determining the difference between the processed output of the network (often a prediction) and a target output. This difference is the error. The network then adjusts its weighted associations according to a learning rule and using this error value. Successive adjustments will cause the neural network to produce output which is increasingly similar to the target output. After a sufficient number of these adjustments the training can be terminated based upon certain criteria. This may also be referred to as supervised learning. Suitable training modes and software are known to a skilled person and described in the Examples below.

According to the present invention, the term "predict" means any statement about a possible outcome or state based on any given analysis. By way of example, a "prediction" in the sense of the present invention may represent an assessment of "predicted" solvent accessible surface residues. The prediction is therefore a computer-implemented assessment of the likelihood of solvent accessibility of any given amino acid in the HLA amino acid sequence. The computer-implemented method or software is capable of determining, according to any given set of parameters, the surface (solvent) accessibility in the computer representation, whereby a prediction is made as to whether the amino acid is indeed accessible in a biological system.

The term "Immune response" in the context of the present invention relates to an immune response as commonly understood by one skilled in the art. An immune response may be understood as a response from a part of the immune system to an antigen that occurs when the antigen is identified as foreign, which preferably subsequently induces the production of antibodies and/or lymphocytes capable of destroying or immobilising the "foreign" antigen or making it harmless. The immune response of the present invention may relate either to a response of the immune system of the recipient against the transplanted material, or an immune response effected by cells of the transplanted cells, tissues, or organs, whereby for example in GVHD T cells of the transplanted material react against and/or attack recipient antigens or tissue. The immune response may be a defense function of the recipient that protects the body against foreign matter, such as foreign tissue, or a reaction of immune cells of the transplanted material against recipient cells or tissue.

The human leukocyte antigen (HLA) system is the major histocompatibility complex (MHC) in humans. The super locus contains a large number of genes related to immune system function in humans. This group of genes resides on chromosome 6, and encodes cell-surface antigen-presenting proteins and has many other functions. The proteins encoded by certain genes are also known as antigens, as a result of their historic discovery as factors in organ transplants. The major HLA antigens are essential elements for immune function. HLAs corresponding to MHC class I (A, B, and C) present peptides from inside the cell (including viral peptides if present). These peptides are produced from digested proteins that are broken down in the proteasomes. In general, these particular peptides are small polymers, about 9 amino acids in length. Foreign antigens attract killer T-cells (also called CD8 positive- or cytotoxic T-cells) that destroy cells. HLAs corresponding to MHC class II (DP, DM, DOA, DOB, DQ, and DR) present antigens from outside of the cell to T-lymphocytes. These particular antigens stimulate the multiplication of T-helper cells, which in turn stimulate antibody-producing B-cells to produce antibodies to that specific antigen.

MHC loci are some of the most genetically variable coding loci in mammals, and the human HLA loci are no exception. Most HLA loci show a dozen or more allele-groups for each locus. Six loci have over 100 alleles that have been detected in the human population. Of these, the most variable are HLA-B and HLA-DRB1. Given the heterodimeric structure of Class II alleles, combinations of alpha and beta chains alter peptide binding specificity, resulting in super-linear peptide binding signatures.

An "allele" is a variant of the nucleotide (DNA) sequence at a locus, such that each allele differs from all other alleles by at least one (single nucleotide polymorphism, SNP) position. Most of these changes result in a change in the amino acid sequences that result in slight to major functional differences in the protein.

"HLA" refers to the human leukocyte antigen locus on chromosome 6p21, consisting of HLA genes (HLA-A, HLA-B, HLA-C, HLA-DRB1, HLA-DQB1 , etc... ) that are used to determine the degree of matching, for example, between a recipient and a donor of a tissue graft. "HLA allele" means a nucleotide sequence within a locus on one of the two parental chromosomes.

"HLA typing" means the identification of an HLA allele of a given locus (HLA-A, HLA-B, HLA-C, HLA-DRB1, HLA-DQB1, etc...). Samples may be obtained from blood or other body samples from donor and/or recipient, which may subsequently be analysed.

Serotyping (identification of the HLA protein on the surface of cells using antibodies) is the original method of HLA typing and is still used by some centres. Phenotyping relates to the serological approach for typing. This method is limited in its ability to define HLA polymorphism and is associated with a risk of misidentifying the HLA type.

Modern molecular methods are more commonly used to genotype an individual. With this strategy, PCR primers specific to a variant region of DNA are used (called PCR SSP). If a product of the right size is found, the assumption is that the HLA allele/ allele group has been identified. PCR-SSO may also be used incorporating probe hybridisation. Reviews of technical approaches towards HLA typing are provided in Erlich H, Tissue Antigens, 2012 Jul;80(1):1-11 and Dunn P, Int J Immunogenet, 2011 Dec;38(6):463-73.

Gene sequencing may be applied, and relates to traditional methods, such as Maxam-Gilbert sequencing, Chain-termination methods, advanced methods and de novo sequencing such as shotgun sequencing or bridge PCR, or the so-called "next-generation" methods, such as massively parallel signature sequencing (MPSS), 454 pyrosequencing, Illumina (Solexa) sequencing, SOLiD sequencing, or other similar methods.

With respect to HLA typing, samples obtained from the donor themselves and/or from donor material before, during or after isolation/preparation for transplantation, may be used for HLA-typing and subsequent comparison of HLA-typing data between subjects. For example, HLA-typing of the donor themselves, for example by analysing a saliva, blood or other bodily fluid sample for HLA information, may occur, and optionally additionally or alternatively, the material obtained from the donor intended for transplantation (donor material) may be analysed for the same and/or complementary HLA type characteristics during HLA-typing.

An "HLA-mismatch" is defined as the identification of different alleles in donor and recipient, which are present at any given loci. Preferably, the HLA mismatch is a difference in protein sequence between two subjects.

According to the present invention, the term "two-field specific HLA protein typing" relates to the standardised HLA naming system developed in 2010 by the WHO Committee for Factors of the HLA System. According to the HLA naming system, the nomenclature is as follows: HLA-Gene*Field1:Field2:Field3:Field4-Suffix. Gene relates to the HLA locus (A, B, C, DRB1, DQB1, etc...). Field 1 relates to the allele group. Field 2 relates to the specific HLA protein. Field 3 relates to a synonymous DNA substitution within the coding region. Field 4 is used to show differences in non-coding regions. The suffix is used to denote particular characteristics in gene expression.

The "two-field" within "two-field specific HLA protein typing" relates to the presence of HLA typing information for Fields 1 and 2 according to the standardized WHO nomenclature used herein. This typing is commonly referred to as "high resolution typing" or "specific HLA protein typing", as the typing provides information on the specific protein sequence relevant for the immune responses dealt with herein.

The term "HLA allele group" typing relates to typing information that is provided for only the HLA gene (locus) and Field1. This typing is commonly referred to as "low resolution" typing. According to the present invention any serotype would be converted to a molecular "low resolution" typing.

The term "intermediate resolution" or "medium resolution" typing relates to some kinds of typing information where the presence of some alleles, or allele strings, have been defined in the patient/donor by the typing method used. The NMDP nomenclature is one method of describing this level of typing. For example A*01:AB relates to alleles 01:01 or 01:02 (01:01/01:02) and the patient/donor is one of these two alleles. The NMDP allele codes are in some cases generic, wherein one code may encompass a number of possible HLA sequences (alleles) at any given HLA locus. Although the donor material may be HLA typed, a number of NMDP codes do not provide information on which specific protein is in fact present at any given HLA locus. This form of typing information is referred to as intermediate resolution typing.

The "donor" is commonly understood to be an individual or multiple individuals (for example in the case of where multiple samples or preparations, such as cord blood units (CBUs) are required for an effective therapeutically relevant amount of donor material for the transplantation) who provide donor material, or in other words biological material, for transplantation in the recipient. References to the donor, or HLA-typing of the donor, may also refer to donor material, or HLA-typing of the donor material, respectively.

The subjects of the invention may be either the donor or recipient, for example where two subjects are compared for HLA mismatches, a donor and recipient are preferably the two subjects.

The subject "recipient" of the method is typically a mammal, preferably a human. The recipient is typically a patient suffering from a disorder characterised by the need for transplantation, such as organ failure necessitating a transplant.

By the term "organ", it is meant to include any bodily structure or group of cells containing different tissues that are organized to carry out a specific function of the body, for example, the heart, lungs, brain, kidney skin, liver, bone marrow, etc. In one embodiment the graft is an allograft, i.e. the individual and the donor are of the same species. The subject may also suffer from a condition that could be treated by the transplantation of cells, even when the disorder itself is not defined by a lack or loss of function of a particular subset of endogenous cells. Some disorders may be treatable by the transplantation of certain kinds of stem cells, whereby the native or endogenous pool of such cells are not necessarily non-functional in the recipient.

The method of the invention is particularly applicable to patients who are about to receive or are predicted to require a transplant, to predict the likelihood of unwanted immune response, such as origin graft damage or rejection, and/or immune origin damage to non-graft tissue. For example, the patient may be expected to receive a transplant in the next one, two, three, four, five, six, or twelve months. Alternatively, the assay is particularly applicable to individuals who have received a transplant to predict the likelihood of immune origin graft damage or rejection, and/or immune origin damage to non-graft tissue. Post-transplant, the method is particularly applicable to patients who show evidence of chronic organ dysfunction (of the graft organ) or possible graft versus host disease (GVHD), particularly chronic GVHD and particularly in cases wherein the graft is a bone marrow transplant.

Transplantation is the moving of biological material, such as cells, tissue or an organ, from one body (donor) to another (recipient or patient), or from a donor site to another location on the patient's own body, or from stored donor material to a recipient's body. Preferably the transplantation is carried out for the purpose of a beneficial medical effect in the recipient, for example by replacing the recipient's damaged or absent organ, or for the purpose of providing stem cells, other cells, tissues or organs capable of providing a therapeutic effect.

Allogeneic transplantation or Allotransplantation is the transplantation of biological material, such as cells, tissues, or organs, to a recipient from a genetically non-identical donor of the same species. The transplant is called an allograft, allogeneic transplant, or homograft. Most human tissue and organ transplants are allografts. Allografts can either be from a living or cadaveric source. Generally, organs that can be transplanted are the heart, kidneys, liver, lungs, pancreas, intestine, and thymus. Tissues include bones, tendons (both referred to as musculoskeletal grafts), cornea, skin, heart valves, nerves and veins.

The invention also encompasses use of the method in the context of screening biological material, such as organs, cells, or tissues produced via regenerative medicine, for example reconstructed donor material that has been constructed ex vivo and is intended for transplantation. Stem cell technologies enable the production of a number of medically relevant cell types or tissues ex vivo. The present invention could therefore also be applied in screening allogeneic material that has been produced by biotechnological and/or tissue engineering methods for its suitability in transplantation.

The invention encompasses the assessment of risk of an immune reaction, preferably a pre-transplantation risk assessment. In embodiments, for stem cells, any given stem cell may be considered as donor material intended for transplantation. For example, hematopoietic stem cell transplantation (HSCT) is the transplantation of multipotent hematopoietic stem cells, usually derived from bone marrow, peripheral blood, or umbilical cord blood. It is a medical procedure common in the fields of hematology and oncology, most often performed for patients with certain cancers of the blood or bone marrow, such as multiple myeloma or leukemia. In these cases, the recipient's immune system is usually destroyed with radiation or chemotherapy before the transplantation. Infection and graft-versus-host disease are major complications of allogenic (also referred to as allogeneic) HSCT.

Stem cells are to be understood as undifferentiated biological cells, that can differentiate into specialized cells and can divide (through mitosis) to produce more stem cells. Highly plastic adult stem cells are routinely used in medical therapies, for example in bone marrow transplantation. Stem cells can now be artificially grown and transformed (differentiated) into specialized cell types with characteristics consistent with cells of various tissues such as muscles or nerves through cell culture. The potential stem cell transplantation may relate to any given stem cell therapy, whereby a number of stem cell therapies exist. Medical researchers anticipate that adult and embryonic stem cells will soon be able to treat cancer, Type 1 diabetes mellitus, Parkinson's disease, Huntington's disease, Celiac disease, cardiac failure, muscle damage and neurological disorders, and many others.

Also known as somatic stem cells and germline stem cells, stem cells can be found in children, as well as adults. Pluripotent adult stem cells are rare and generally small in number but can be found in a number of tissues including umbilical cord blood. Bone marrow has been found to be one of the rich sources of adult stem cells which have been used in treating several conditions including Spinal cord injury, Liver Cirrhosis, Chronic Limb Ischemia and End-stage heart failure. Adult stem cells may be lineage-restricted (multipotent) and are generally referred to by their tissue origin (mesenchymal stem cell, adipose-derived stem cell, endothelial stem cell, dental pulp stem cell, etc.).

Multipotent stem cells are also found in amniotic fluid. These stem cells are very active, expand extensively without feeders and are not tumorigenic. Amniotic stem cells are multipotent and can differentiate in cells of adipogenic, osteogenic, myogenic, endothelial, hepatic and also neuronal lines. It is possible to collect amniotic stem cells for donors or for autologuous use.

Cord blood-derived multipotent stem cells display embryonic and hematopoietic characteristics. Phenotypic characterization demonstrates that (CB-SCs) display embryonic cell markers (e.g., transcription factors OCT-4 and Nanog, stage-specific embryonic antigen (SSEA)-3, and SSEA-4) and leukocyte common antigen CD45, but that they can be negative for blood cell lineage markers. Additionally, CB-SCs display very low immunogenicity as indicated by expression of a very low level of major histocompatibility complex (MHC) antigens and failure to stimulate the proliferation of allogeneic lymphocytes.

HSC are typically available from bone marrow, Peripheral blood stem cells, Amniotic fluid, or Umbilical cord blood. In the case of a bone marrow transplant, the HSC are removed from a large bone of the donor, typically the pelvis, through a large needle that reaches the center of the bone. The technique is referred to as a bone marrow harvest and is performed under gen-eral anesthesia. Peripheral blood stem cells are a common source of stem cells for allogeneic HSCT. They can be collected from the blood through a process known as apheresis. The donor's blood is withdrawn through a sterile needle in one arm and passed through a machine that removes white blood cells. The red blood cells may be returned to the donor. The peripheral stem cell yield may be boosted with daily subcutaneous injections of Granulocyte-colony stimulating factor, serving to mobilize stem cells from the donor's bone marrow into the peripheral circulation.

It is also possible to extract hematopoietic stem cells from amniotic fluid. Umbilical cord blood is obtained from an infant's Umbilical Cord and Placenta after birth. Cord blood has a higher concentration of HSC than is normally found in adult blood. However, the small quantity of blood obtained from an Umbilical Cord (typically about 50 ml.) makes it more suitable for transplantation into small children than into adults. Multiple units could however be used. Newer techniques using ex-vivo expansion of cord blood units or the use of two cord blood units from different donors allow cord blood transplants to be used in adults. Cord blood can be harvested from the umbilical cord of a child being born.

Unlike other organs, bone marrow cells can be frozen (cryopreserved) for prolonged periods without damaging too many cells. This is a necessity with autologous HSC because the cells are generally harvested from the recipient months in advance of the transplant treatment. In the case of allogeneic transplants, fresh HSC are preferred in order to avoid cell loss that might occur during the freezing and thawing process. Allogeneic cord blood is typically stored frozen at a cord blood bank because it is only obtainable at the time of childbirth. To cryopre-serve HSC, a preservative, DMSO, must be added, and the cells must be cooled very slowly in a controlled-rate freezer to prevent osmotic cellular injury during ice crystal formation. HSC may be stored for years in a cryofreezer, which typically uses liquid nitrogen. In light of this, the invention may relate to typing and risk assessment of donor material already stored as described herein, before being considered for transplantation.

The invention encompasses the assessment of risk of an immune reaction, preferably a pre-transplantation risk assessment, whereby any given organ or tissue may be considered as donor material intended for transplantation. For example, kidney transplantation or renal transplantation is the organ transplant of a kidney into a patient, for example with end-stage renal disease. Kidney transplantation is typically classified as deceased-donor (formerly known as cadaveric) or living-donor transplantation depending on the source of the donor organ. Living-donor renal transplants are further characterized as genetically related (living-related) or non-related (living-unrelated) transplants, depending on whether a biological relationship exists between the donor and recipient.

Alloreactivity is defined as the reaction of a lymphocyte or antibody with an alloantigen, which may be understood as an antigen from foreign material. Alloantigen recognition may occur via direct or indirect alloantigen recognition, by which T cells may recognize alloantigens and potentially lead to transplant rejection after an organ transplantation. Furthermore, alloantigen recognition may occur via T cell dependent or T cell independent recognition, by which B cells may recognize alloantigens and potentially lead to transplant rejection after an organ transplantation. In T cell dependent B cell activation, an immune response is triggered and reinforced by a conformational loop between B cells' alloantigen presentation and T cell activation. This concept is described as "linked recognition".

Graft-versus-host disease (GVHD) is a relatively common complication following an allogeneic cell, tissue or organ transplant. It is commonly associated with stem cell or bone marrow transplant but the term also applies to other forms of tissue graft or organ transplant. Immune cells (typically white blood cells) in the tissue (the graft) recognize the recipient (the host) as "foreign". The transplanted immune cells then attack the host's body cells. GVHD can also occur after a blood transfusion if the blood products used have not been irradiated.

The method, system or computer product of the invention may in some embodiments comprise one or more conventional computing devices having a processor, an input device such as a keyboard or mouse, memory such as a hard drive and volatile or nonvolatile memory, and computer code (software) for the functioning of the invention. The computers may also comprise a programmable printed circuit board, microcontroller, or other device for receiving and processing data signals such as those received from the local controllers, programmable manufacturing equipment, programmable material handling equipment, and robotic manipulators.

The method, system or computer product may comprise one or more conventional computing devices that are pre-loaded with the required computer code or software, or it may comprise custom-designed hardware. The system may comprises multiple computing devices which perform the steps of the invention. In certain embodiments, a plurality of clients such as desktop, laptop, or tablet computers can be connected to a server such that, for example, multiple users can enter their orders for personalized products at the same time. The computer system may also be networked with other computers over a local area network (LAN) connection or via an Internet connection. The system may also comprise a backup system which retains a copy of the data obtained by the invention. The data connections of step e) may be conducted or configured via any suitable means for data transmission, such as over a local area network (LAN) connection or via an Internet connection, either wired or wireless.

A client or user computer can have its own processor, input means such as a keyboard, mouse, or touchscreen, and memory, or it may be a dumb terminal which does not have its own independent processing capabilities, but relies on the computational resources of another computer, such as a server, to which it is connected or networked. Depending on the particular implementation of the invention, a client system can contain the necessary computer code to assume control of the system if such a need arises. In one embodiment, the client system is a tablet or laptop.

The components of the computer system may be conventional, although the system may be custom-configured for each particular implementation. The computer system may run on any particular architecture, for example, personal/microcomputer, minicomputer, or mainframe systems. Exemplary operating systems include Apple Mac OS X and iOS, Microsoft Windows, and UNIX/Linux; SPARC, POWER and Itanium-based systems; and z/Architecture. The computer code to perform the invention may be written in any programming language or model-based development environment, such as but not limited to C/C++, C#, Objective-C, Java, Basic/VisualBasic, MATLAB, Simulink, StateFlow, Lab View, or assembler. The computer code may comprise subroutines which are written in a proprietary computer language which is specific to the manufacturer of a circuit board, controller, or other computer hardware component used in conjunction with the invention.

The method, system or computer product, and database of the invention, can employ any kind of file format which is used in the industry. For example, a digital representation of a protein sequence, protein structure, or other computer-implemented aspect of the invention, can be stored in a proprietary format, DXF format, XML format, or other format for use by the invention. Any suitable computer readable medium may be utilized. The computer-usable or computer-readable medium may be, for example but not limited to, an electronic, magnetic, optical, electromagnetic, infrared, or semiconductor system, apparatus, device, or propagation medium. More specific examples (a non-exhaustive list) of the computer-readable medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a transmission media such as those supporting the Internet or an intranet, cloud storage or a magnetic storage device.

Embodiments of the present invention are described below with reference to flowchart figures and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions, such as by modules. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

### FIGURES

The following figures are presented to demonstrate potentially preferred aspects or embodiments of the invention in a practical context. The figures are as such not limiting to the scope of the invention.

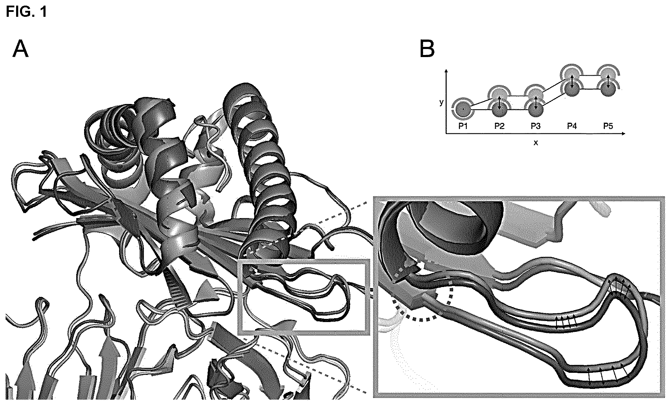

**Fig 1:** (A) Cartoon plot of 3MRE (pink, HLA14697) and 3GSR (petrol, HLA 14697, superimposition based on identical amino acid sequences). Blue box indicates small changes in dihedral angles (purple circle) causing large Euclidean distances in the following loop structure. (B) Euclidean distance between atom positions may overestimate the structural difference (P3-P5) due to distance-propagation due to distant angular difference at P1/P2 or suboptimal superimposition. However, dihedral angles at P3-P5 are identical, only considering P1/P2 as structurally different.

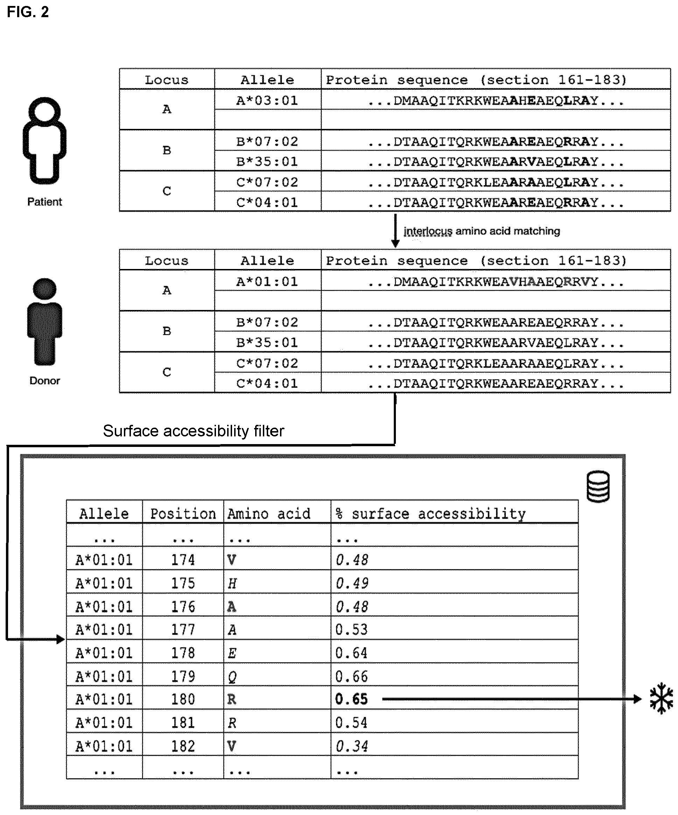

**Fig 2****:** Snowflake HLA matching algorithm schematic. Donor amino-acid mismatches with a predicted threshold-exceeding allele- and position-specific solvent accessibility increase the Snowflake score by one.

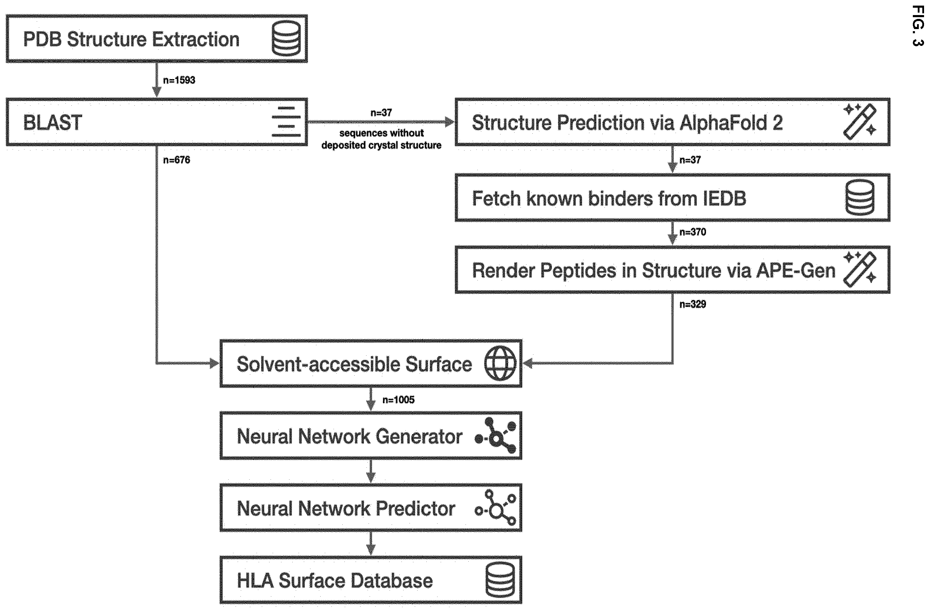

**Fig 3****:** Snowflake surface accessibility prediction pipeline.

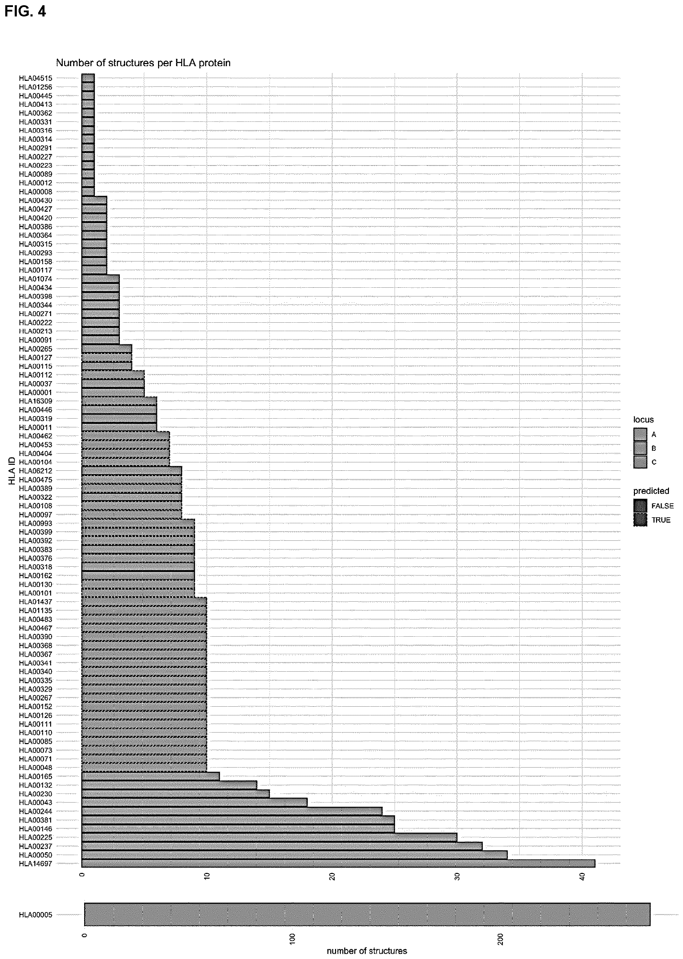

**Fig 4****:** Bar chart indicating the distribution of alleles in experimental structures. Color indicates locus, dashed border indicates structure prediction.

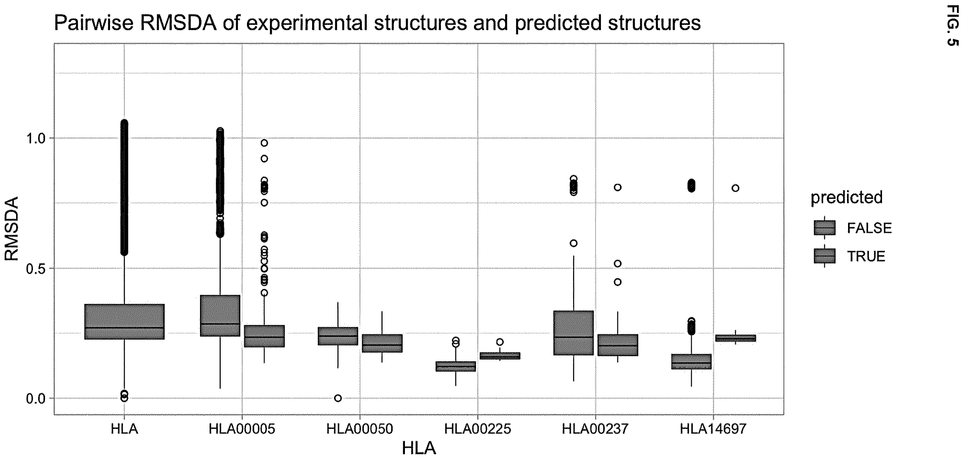

**Fig 5****:** Boxplot indicating the pairwise root mean square dihedral angle differences (RMSDA) across all experimental HLA Class I structures and pairwise across the most common HLA Class I structures, including their corresponding predicted structure representation.

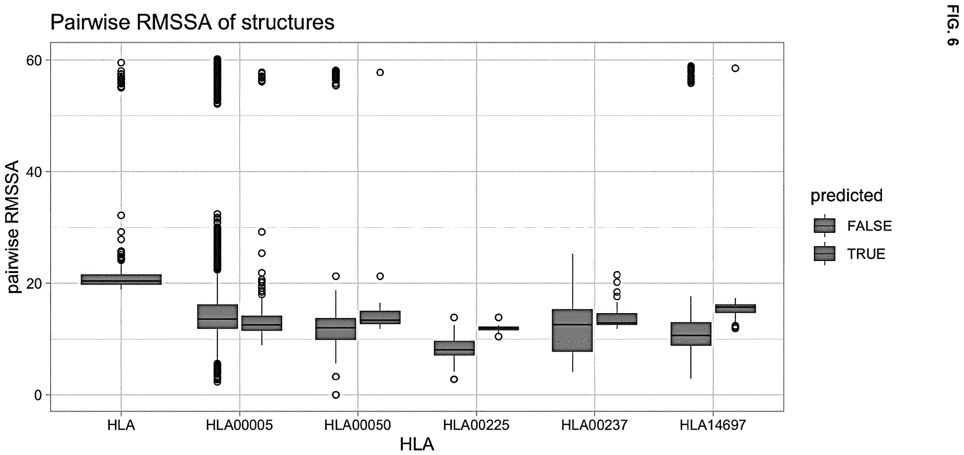

**Fig 6****:** Boxplot indicating the pairwise root mean square surface area differences (RMSSA) across all experimental HLA Class I structures and pairwise across the most common HLA Class I structures.

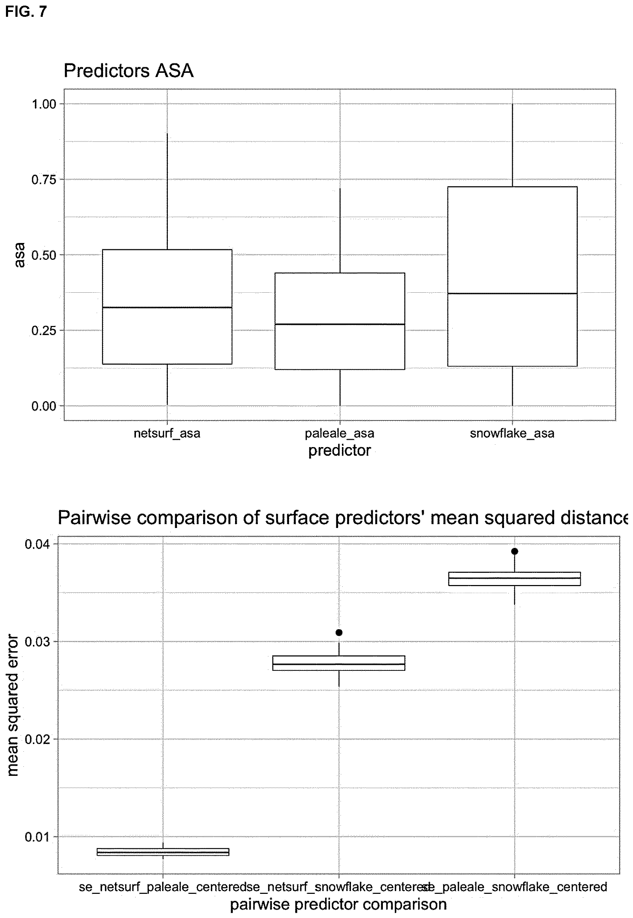

**Fig 7****:** (A) Solvent accessibility distribution of reference alleles as predicted by NetSurfP (i.e. "rsa"), PaleAle (i.e. Relative Solvent Accessibility) and Snowflake. (B) Pairwise mean squared distance between centered predictions of NetSurfP and PaleAle, NetSurfP and Snowflake, and PaleAle and Snowflake.
**Fig 8****:** Pairwise comparison of predictions considering reference alleles with respective predictor's mean as cutoff for surface accessibility.
**Fig 9****:** Highlighted amino acid positions of selected structures. HLA alpha chain is colored in petrol, beta-2-microglobulin is colored in green, the bound peptide is colored in blue, and the highlighted amino acid positions are colored pink. (A) Position P167 is predicted by Snowflake and the reference predictors to be buried in A*01:01. (B) The same position P167 in A*02:01 is predicted by Snowflake to be accessible, by the reference predictors again predicted to be buried. (C) The reference predictors estimate position P110 in A*02:01 to be buried, whilst Snowflake considers it accessible. (D) For A*02:01, positions P119 and P120 are contact points with the Beta-2-microglobulin. Whilst Snowflake considers these positions not being accessible, NetSurfP considers them as accessible.

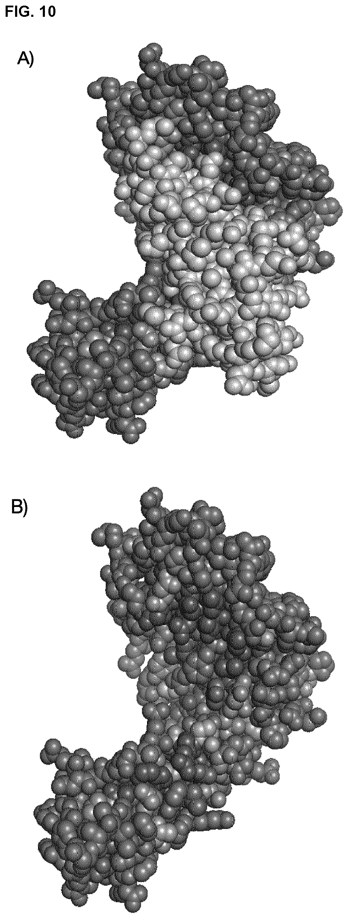

**Fig 10****:** (A) Solvent accessibility-colored residues of A*02:01. (B) Residues masked by the beta-2-microglobulin structure are predicted to be inaccessible. Color based on Snowflake score, red tones indicating higher solvent accessibility, blue tones indicate lower solvent accessibility.

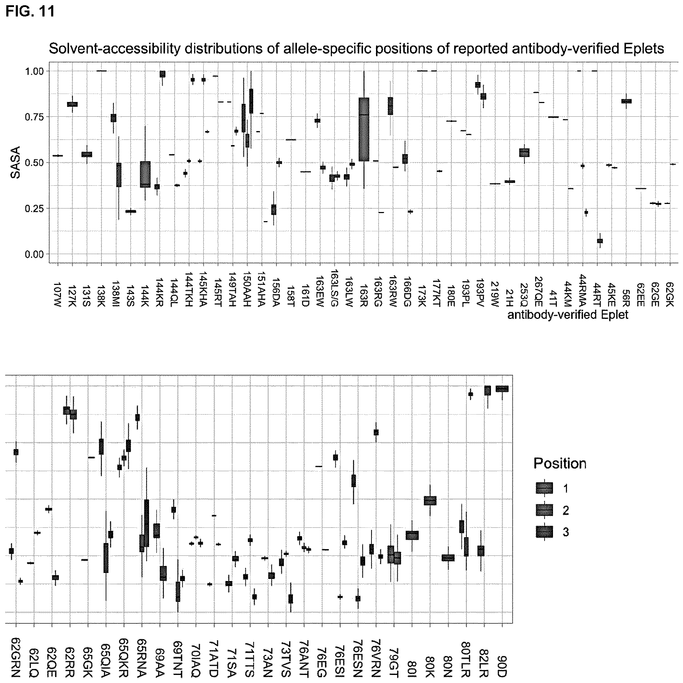

**Fig 11****:** Distributions of solvent accessibility of respective antibody-verified Eplets positions. Color corresponds to the amino acid position within the Eplet.

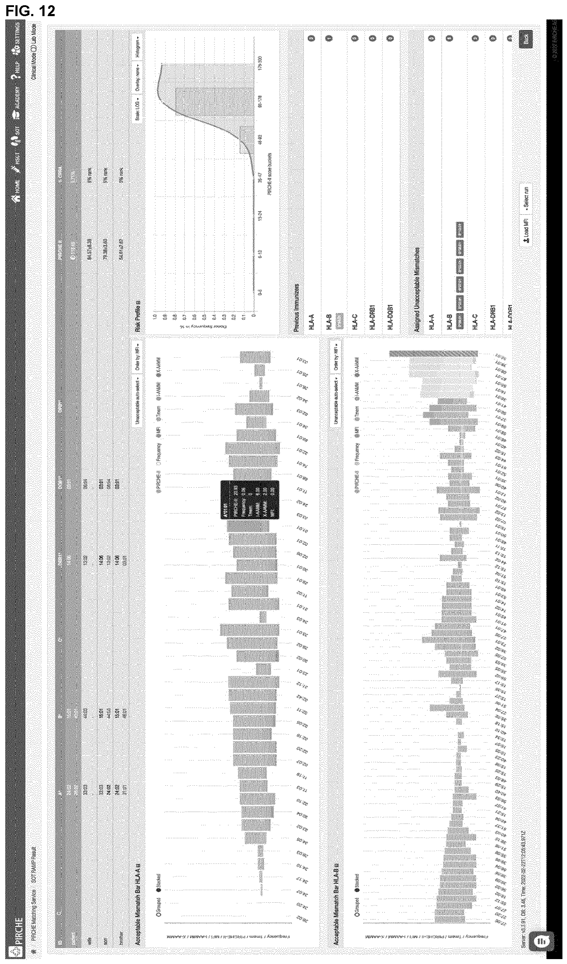

**Fig 12****:** PIRCHE Risk and Acceptable Mismatch Profile including PIRCHE-II epitope scores (blue bars), mean fluorescence intensities of imported single antigen bead assays (pink bars), shared T cell epitopes with previous immunizers (orange bars), allele frequencies (pale green bars), interlocus Snowflake scores (purple bars) and intralocus Snowflake scores (dark green bars).

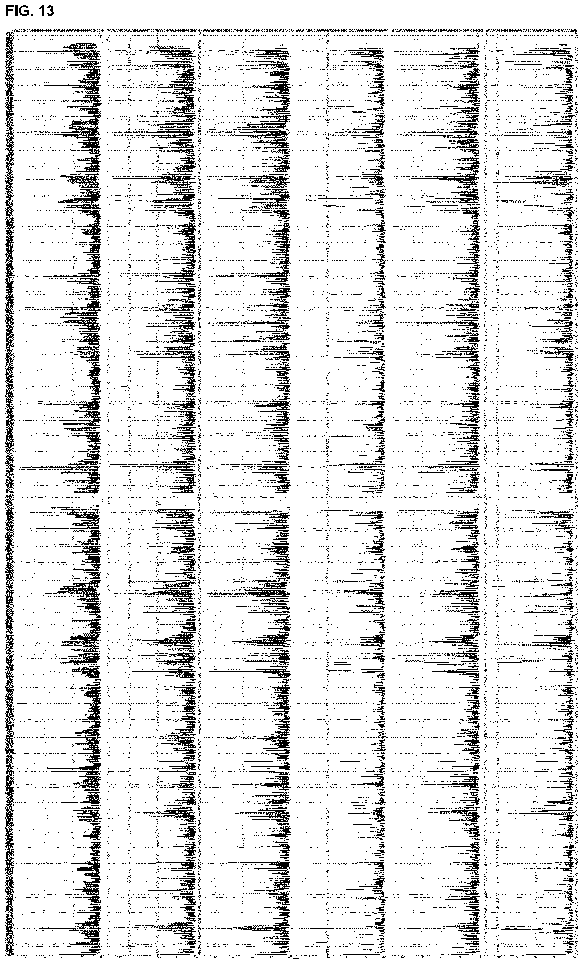

**Fig. 13****:** Position-specific square dihedral angle difference (SDA) in phi/psi angles (vertical columns). Top row indicating all experimental HLA Class I structures, following rows indicating the alleles with the most structural datasets in the PDB (HLA00005, HLA00050, HLA00225, HLA00237, HLA14697). Green boxes depict pairwise SDA of experimental structures, red boxes depict pairwise SDA with the predicted structure.

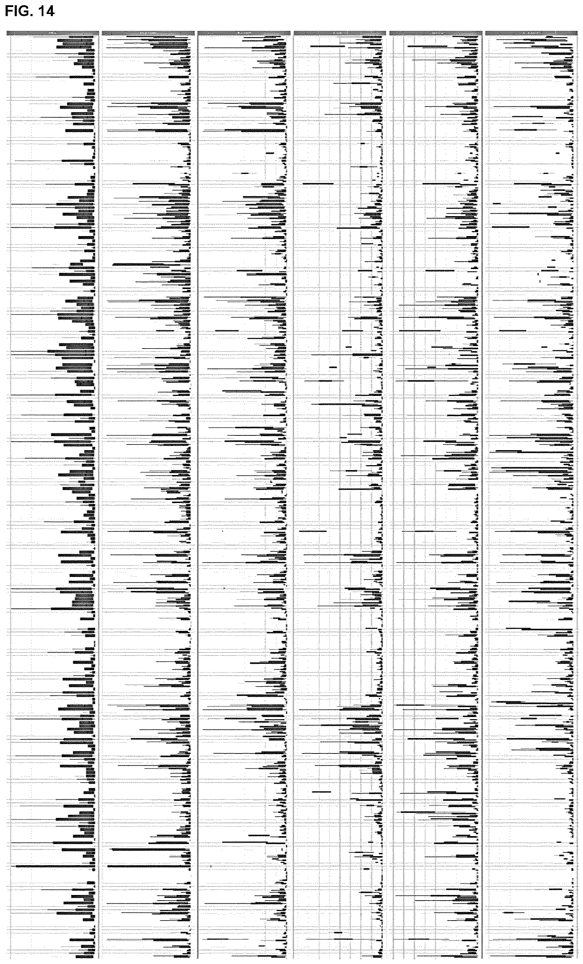

**Fig. 14****:** Position-specific square surface area difference (SSA). Top row indicating all experimental HLA Class I structures, following rows indicating the alleles with the most structural datasets in the PDB (HLA00005, HLA00050, HLA00225, HLA00237, HLA14697). Green boxes depict pairwise SSA of experimental structures, red boxes depict pairwise SDA with the predicted structure.

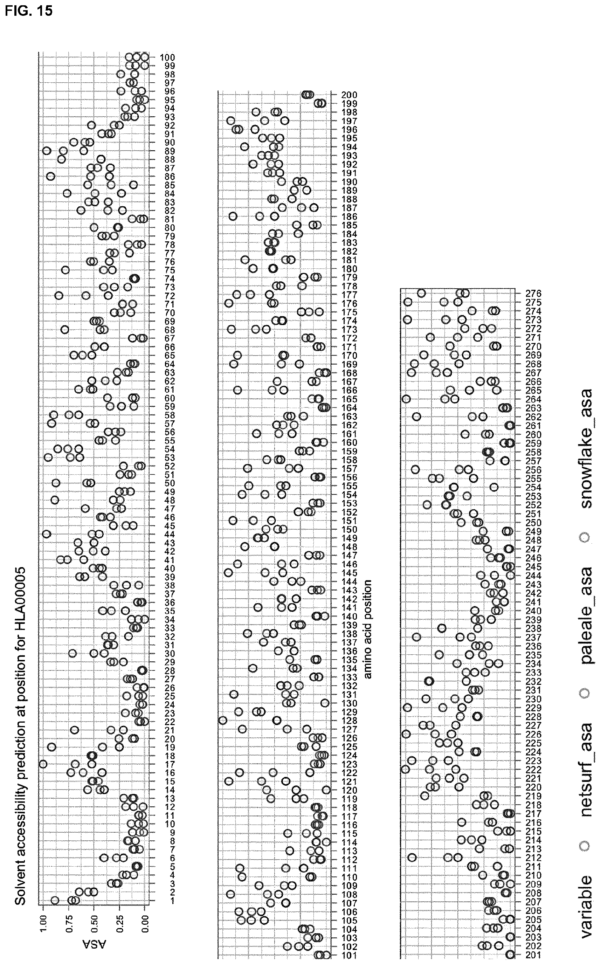

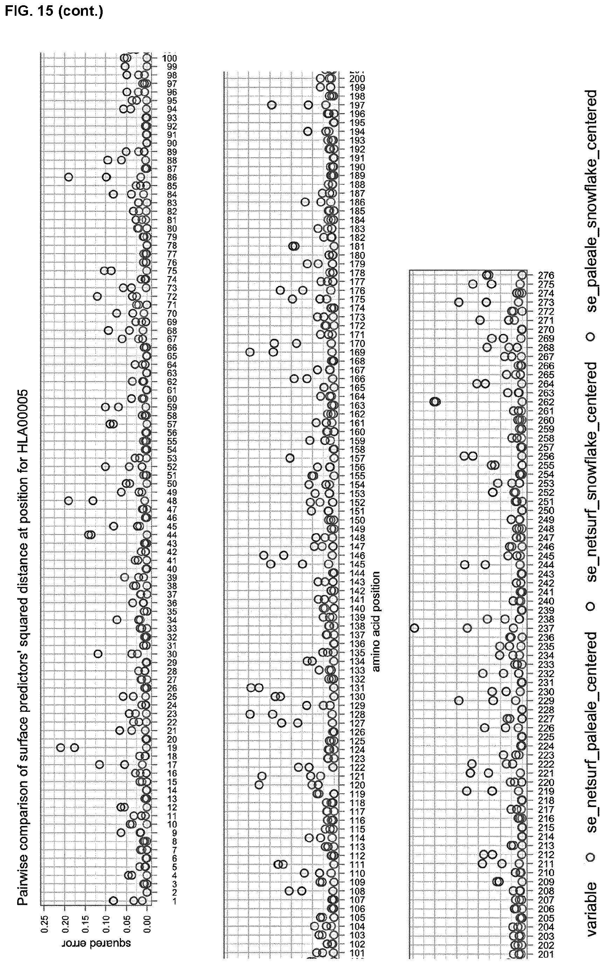

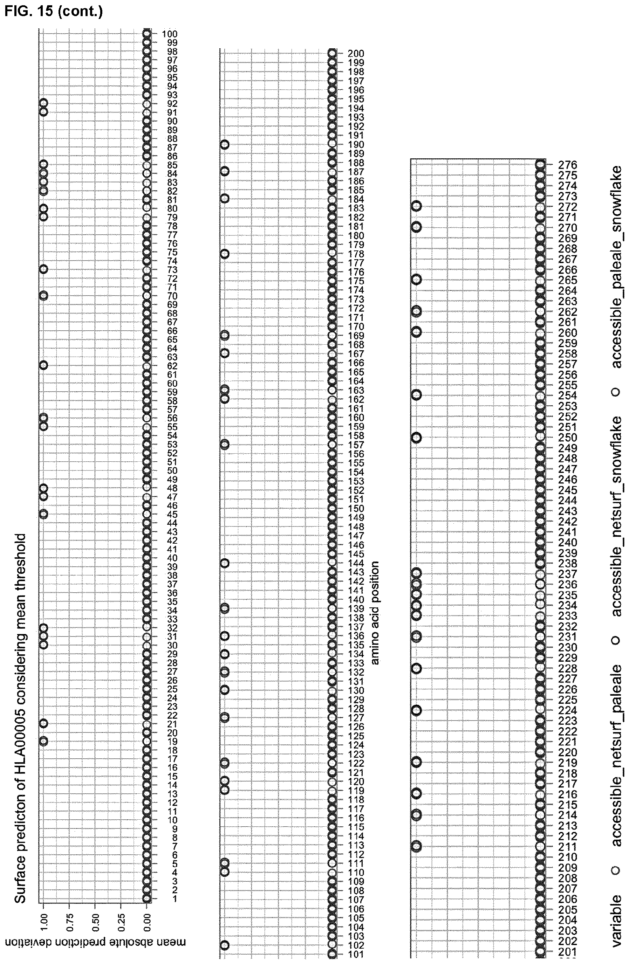

**Fig. 15****:** (A) Position-specific solvent accessibility prediction for HLA00005 (i.e. A*02:01). Color depicts the respective predictor. (B) Position-specific pairwise squared distance between the predictors' centered prediction. Higher values indicate stronger disagreement between the predictors. Color depicts the respective compared predictor pair. (C) Position-specific pairwise disagreement considering the respective predictor's mean as a threshold for surface accessibility. Scores at zero indicate agreement between predictors, scores at one indicate disagreement between the predictors. Color depicts the respective compared predictor pair.

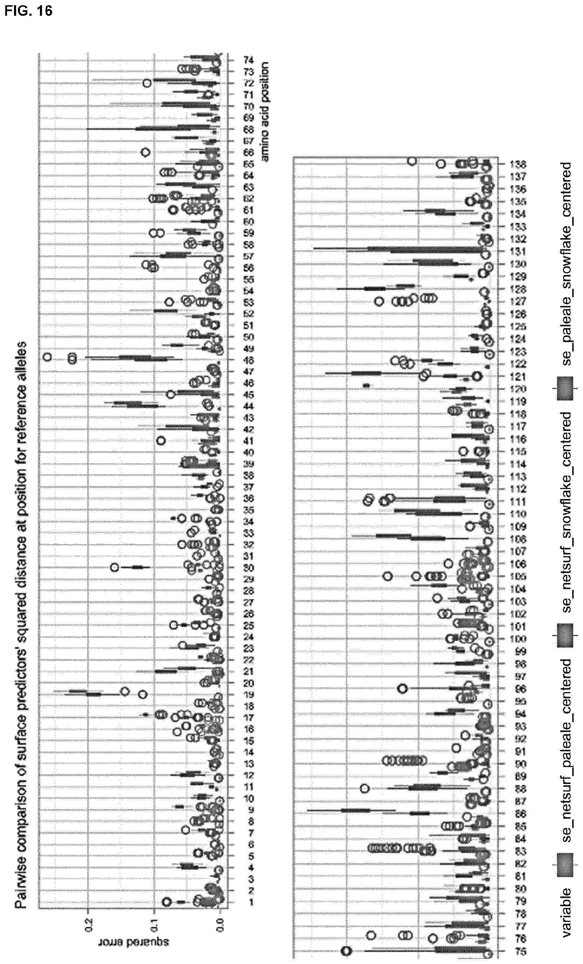

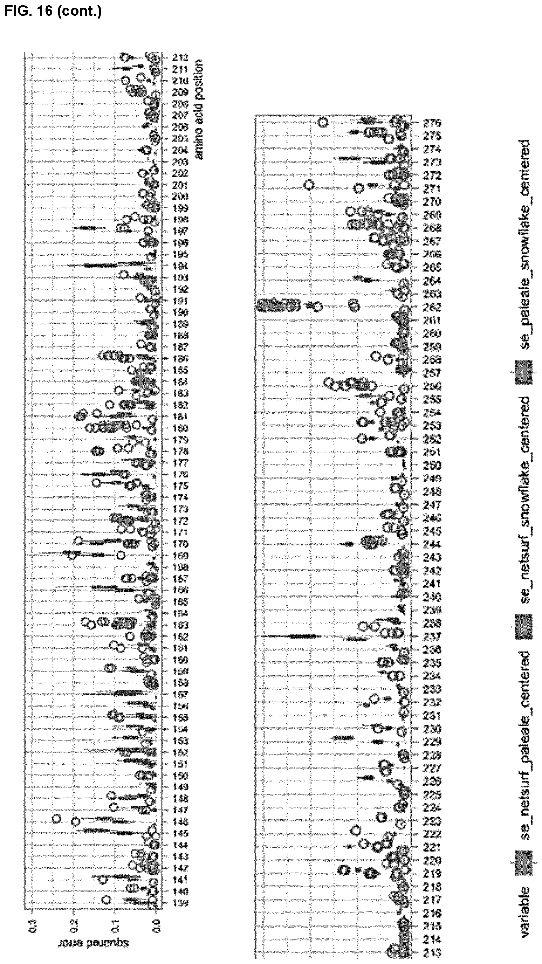

**Fig. 16****:** Position-specific squared error of pairwise predictors' solvent accessibility score comparisons considering 72 reference alleles. Red boxes/circles: NetSurfP vs. PaleAle, green boxes/circles: NetSurfP vs. Snowflake, blue boxes/circles: PaleAle vs. Snowflake, circles: outliers.

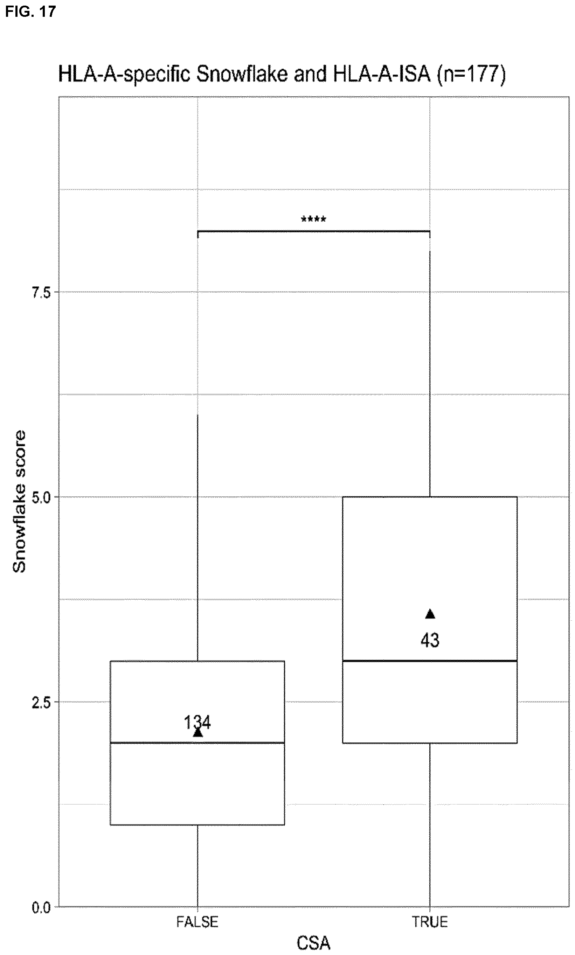

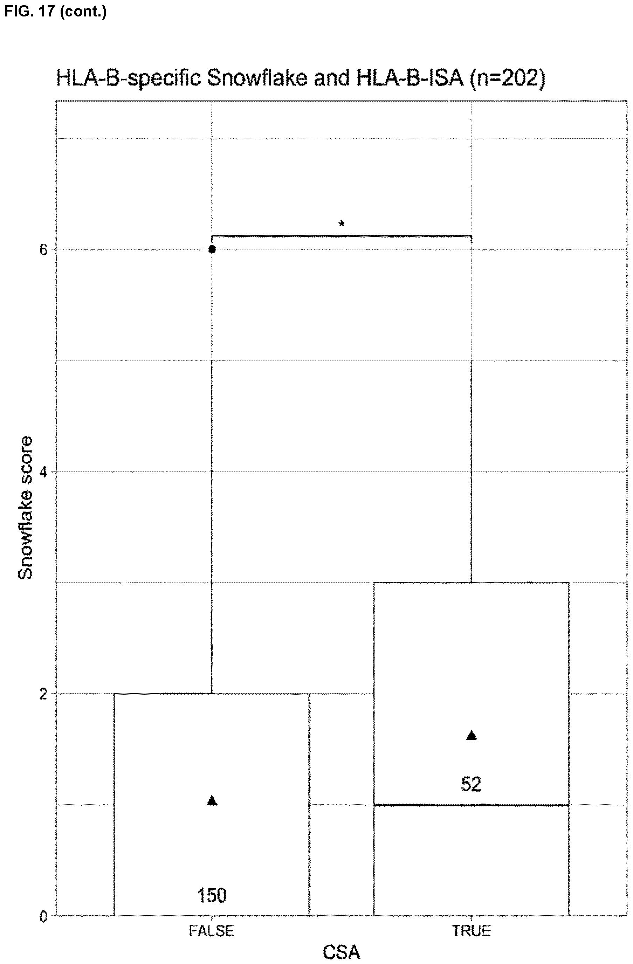

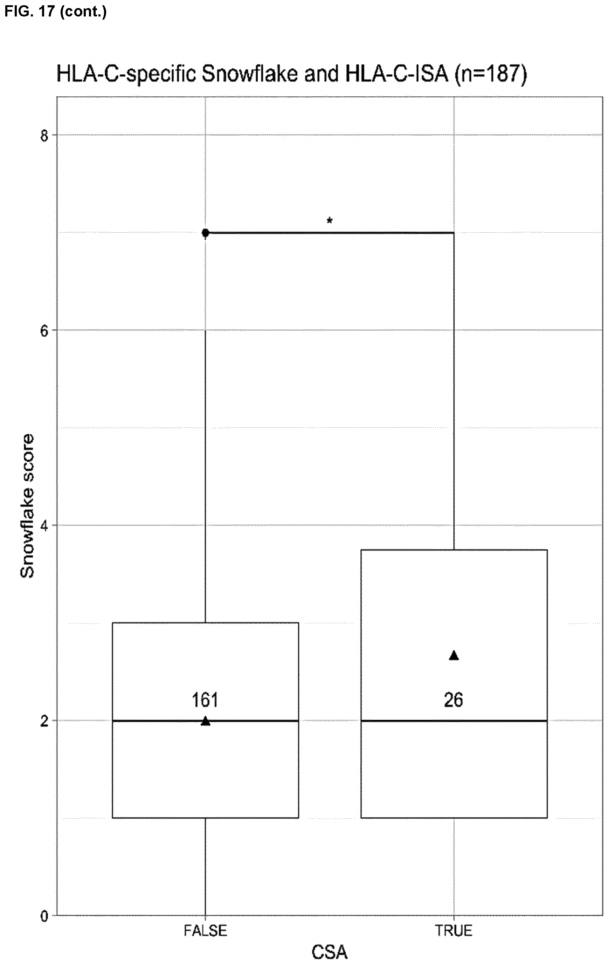

**Fig 17****:** Snowflake score distribution of locus-specific HLA mismatches resulting in paternal mismatch-specific antibodies for (A) HLA-A, (B) -B and (C) -C. Red labels indicate the number of cases per box. Boxplots depict the median (horizontal line), mean (triangle), and first to third quartile (box); the highest and lowest values within 1.5x IQR (whiskers) and outliers (circles), respectively. *, p ≤ 0.05; ****, p ≤ 0.0001; HLA, human leukocyte antigen; ISA, immunizer-specific antibodies; CSA, child-specific HLA antibody; IQR, interquartile range.

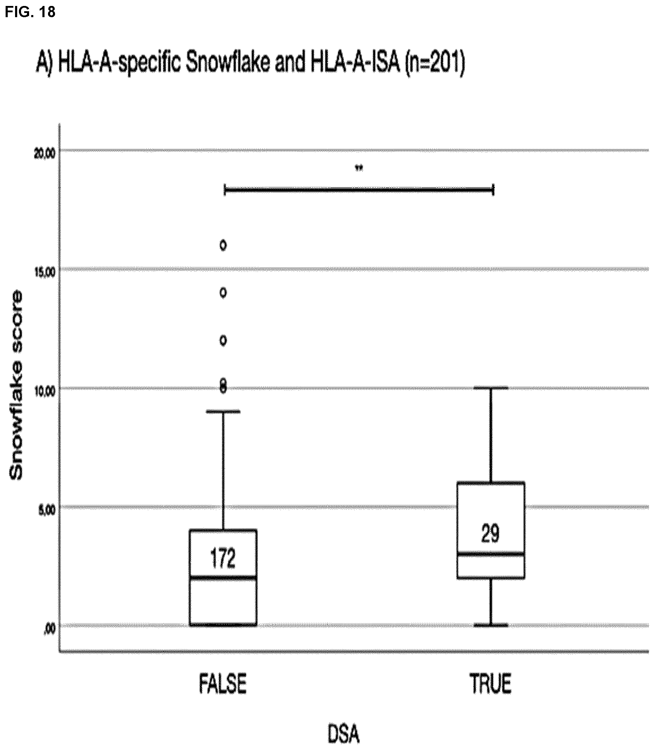

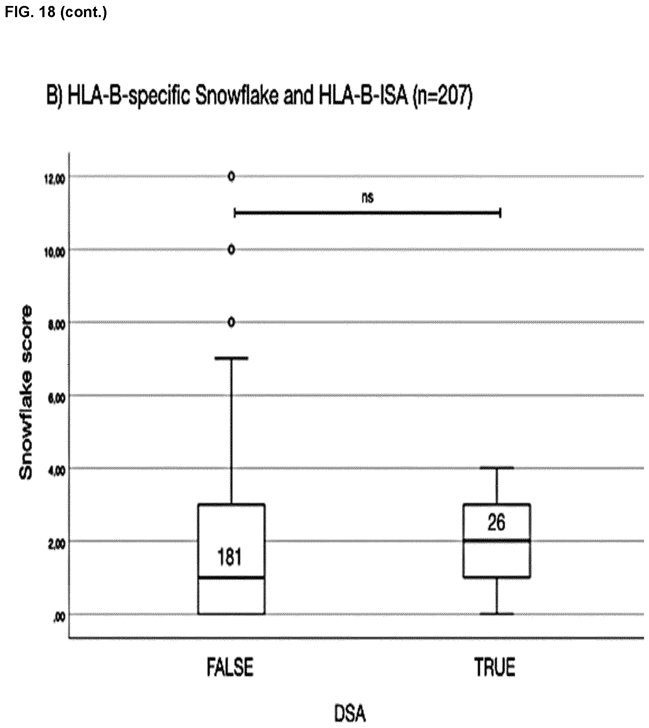

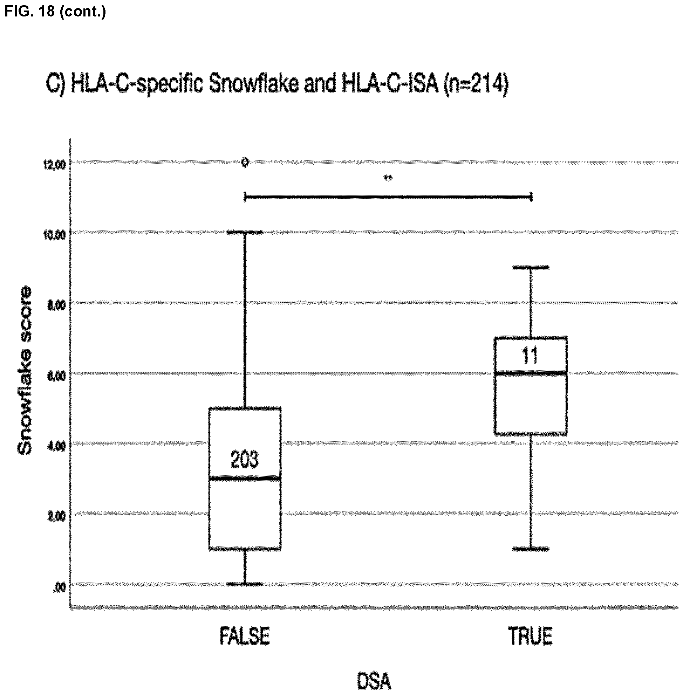

**Fig 18****:** Snowflake score distributions depending on HLA locus and presence of donor-specific antibodies (DSA). (A) HLA-A-specific Snowflake score in A-specific DSA negative/positive patients. (B) HLA-B-specific Snowflake score in B-specific DSA negative/positive patients. (C) HLA-C-specific Snowflake score in C-specific DSA negative/positive patients. Red labels indicate the number of cases per box. Boxplots depict the median (horizontal line), and first to third quartile (box); the highest and lowest values within 1.5x IQR (whiskers) and outliers (circles, rhombi), respectively. **, p ≤ 0.01; ns, p > 0.05; HLA, human leukocyte antigen; ISA, immunizer-specific antibodies; DSA, donor-specific HLA antibody; IQR, interquartile range.

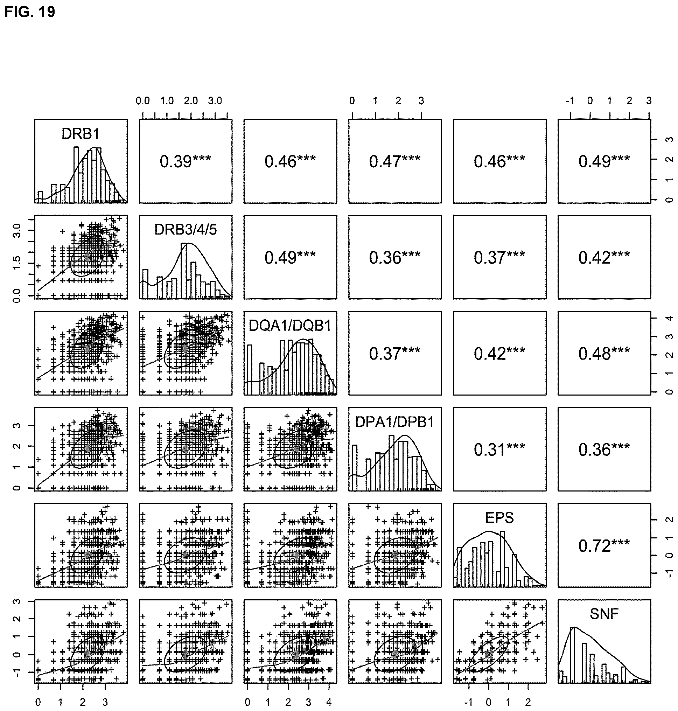

**Fig 19****:** Correlation scatter matrix of scaled (EPS, SNF) and logarithmic (PIRCHE-DRB1, PIRCHE-DRB3/4/5, PIRCHE-DQA1/DQB1, PIRCHE-DPA1/DPB1) epitope match scores in the PC. Numbers indicate Spearman's rho (rs). ***: p < 0.001.

### EXAMPLES

The following examples are presented to demonstrate potentially preferred aspects or embodiments of the invention in a practical context. The examples are as such not limiting to the scope of the invention.

For the purpose of these Examples, the term "Snowflake" represents an exemplary embodiment of the inventive methods.

In Example 1 the Snowflake B cell epitope matching algorithm is presented, a deep-learning based software evaluating allele-specific surface-accessible amino acid mismatches.

Clinical studies are presented in Example 2, indicating a correlation between the Snowflake score and immunological events, such as development of donor-specific antibodies.

### Example 1: Snowflake: A deep learning-based human leukocyte antigen matching algorithm considering allele-specific surface accessibility.

### Methods:

### Extraction of experimental structures from PDB

HLA protein structures were extracted from the Protein Data Bank (PDB, www.rcsb.org) [16] using the full text search term "hla". The found structures were filtered by structures comprising chains "A", "B" and "C", whereas chain B's amino acid sequence was expected to match Beta-2-microglobulin (UniprotKB entry P61769). The supposed alpha chains were aligned to the IPD/IMGT-HLA database [17] (version 3.46.0) using BLAST [18] via the Biopython library [19]. Structures were only considered further, if a BLAST score of 1300 was exceeded. HLA allele names were assigned based on the maximum number of identical amino acid configurations and lowest HLA identifier in case of a tie.

### Structural prediction of non-crystallized HLA proteins

Given the large number of known HLA genes and the complexity of generating structural data experimentally, the AlphaFold [13-15] protein structure inference pipeline (v2.0) was applied to supplement PDB data. As the public AlphaFold Database does not cover HLA variants, the AlphaFold software was installed on an Amazon Web Services Elastic Cloud Compute server to run de novo predictions. Generating HLA protein 3D structures via AlphaFold is a heavy computational task. Consequently, HLA alleles were selected for structure prediction, whose serologic and antigenic groups were not covered by structures from the PDB. For visualization, all structures were superimposed as described by [20] via the Biopython library [19].

### Render peptide in predicted structures' binding groove

The inferred structures comprise the proteins' respective alpha chain and the Beta-2-microglobulin, but lack a bound peptide, which alters the solvent accessibility for residues involved in the peptide-binding domain. Despite AlphaFolds high prediction accuracy and capability to infer multimeric structures, it is not designed to predict binding of antibodies [15]. To supplement the predicted structures with peptides, the Immune Epitope Database (IEDB, www.iedb.org) [21] was screened for nonameric peptides being reported as binders to the respective HLA protein. For predicted alleles without reported binders, the IEDB-reported peptides of the allele with the highest amino acid identity in the extracellular domains were considered as binders. For each allele, a subset of ten peptides was extracted and each peptide's position in the HLA binding groove was predicted by the Anchored Peptide-MHC Ensemble Generator (APE-Gen) [22].

### Structure comparison

The position-specific square of dihedral angle differences (SDA) and the root mean SDA (RMSDA) were calculated as superimposition-independent measures of similarity of identical amino acid sequence structures (as reviewed by [23], Fig A). Additionally, the root mean square of solvent accessible surface area (RMSSA) was calculated across all residues. This metric was furthermore applied to sequence-mismatched proteins. Wilcoxon signed rank test with Bonferroni correction was applied to evaluate significance of dissimilarity.

### Calculation of protein-residue-specific surface area

For all experimental and inferred HLA protein structures, each residues' solvent accessible surface area was calculated considering the Shrake-Rupley rolling-probe algorithm [24], implemented by the Biotite library [25]. Surface area computation considered the proteins' alpha chain, Beta-2-microglobulin and bound peptide. The probe size was set to 1.4 Å corresponding to the radius of water molecules.

### Neural network design

The experimental and inferred HLA structures comprise only a fraction of known HLA Class I proteins. To create a database of surface accessible residues for all HLA Class I proteins, a long short-term memory bidirectional recurrent neural network [26] was implemented, that considers the HLA alpha chains' amino acid configurations as inputs and the residues' surface areas as output. The network configuration stacked a bidirectional layer with rectified linear unit (ReLU) activation of 100 neurons, a second bidirectional layer with 64 ReLU neurons, a third bidirectional layer with 32 ReLU neurons and the output layer considering a linear activation function. Position-specific surface area was limited to 100. The model-generation and inference was implemented in the Python programming language (Python Software Foundation, version 3.5.1) using Keras (https://keras.io) / Tensorflow 2 (https://www.tensorflow.org).

### Comparison of calculated surface area with other methods

The performance of the applied surface prediction pipeline was evaluated by comparing the predicted surface accessibility of 72 IMGT-IPD/HLA-reported HLA Class I reference alleles (based on IMGT 3.46.0, [27]) with the predictions provided by NetSurfP 2.0 [11] and PaleAle 4.0 [12]. Relative solvent accessibility predicted by PaleAle was divided by 100 to match the other predictors' outputs. The produced relative solvent accessibility scores were mean-centered. Position-specific squared error (SE) and mean squared errors (MSE) were calculated pairwise between the predictors and reference alleles. Amino acid sequence positions with large SE were visualized in PyMol 2.3.0. Amino acid positions with solvent accessibility scores exceeding the respective predictor's mean solvent accessibility were considered accessible and inaccessible otherwise.

### Epitope matching based on surface-accessible amino acid residues

The proposed method considers donor amino acid mismatches at allele-specific positions exceeding a surface accessibility threshold (i.e. are predicted to be surface-accessible) between two individuals as epitope mismatches (Fig 2). The sum of such amino acid mismatches is considered the Snowflake epitope mismatch score. The algorithm is parameterized in the surface accessibility threshold and the mode of reference loci: (1) intralocus matching considers only the same recipient locus, whereas (2) interlocus matching references all recipient's HLA Class I proteins.

### Statistical analysis

All calculations were executed in R software (R 3.6.1, R Foundation for Statistical Computing, Vienna, Austria).

### Results

The PDB search yielded 1593 structures that were filtered down to 676 HLA Class I experimental structures (Fig 3). The majority of experimental HLA Class I structures correspond to the HLA00005 (i.e. A*02:01, n=277) (Fig 4). Based on these structures, additional 37 Class I alleles (14 HLA-A, 16 HLA-B, 7 HLA-C) were selected for structure prediction.

The distribution of pairwise RMSDA for the most common structures is similar compared to the overall RMSDA distribution considering structural variance across all HLA. Predicted structures RMSDA with their corresponding experimental structures is similar to the RMSDA deviation between experimental structures of the same allele (Fig 5). It is observed that certain regions of the HLA have higher SDA compared to rigid regions with low SDA (Fig 13).

There is considerable variance in RMSSA observed within the various reported experimental structures of the same allele. The RMSSA across all HLA Class I experimental structures is however significantly higher compared to the allele-specific RMSSAs, indicating a higher variance between individual alleles (Fig 6). Also for the position-wise variance of surface area, certain regions of the HLA are subject to higher variance in SSA than others (Fig 14)

### Compare surface prediction with reference tools

After training the Snowflake surface predictor in 400 epochs, the loss function converged to ~0.02. The average predicted relative solvent accessibility ranged lower for NetSurfP and PaleAle (i.e. reference predictors) compared to Snowflake (Fig 7A). After centering, the reference predictors produced similar predictions with low MSE across reference alleles (Fig 7B). Snowflake had considerably higher MSEs considering the reference predictors (Fig 7B).

The low MSE between NetSurfP and PaleAle was also observed in the low position-specific SE (Fig 16) at the majority of amino acid positions. Position-specific SE for Snowflake was considerably higher in certain regions (Fig 16). Classifying solvent accessibility by exceeding the respective predictor's median score yielded a considerable number of allele-dependent (e.g P3, P110, P190) and allele-independent (e.g P31, P167 or P219) deviations between the Snowflake and the reference predictors (Fig 8). As an example, the solvent accessibility prediction and SE of A*02:01 were provided in Figure 15.

Generating visual representations of some of these characteristic positions indicates allele-specific differences in surface prediction (Fig 9). Amino acid position 167 of A*01:01 is not predicted to be solvent-accessible by Snowflake, PaleAle and NetSurfP. Visually these predictions appear plausible. Despite PaleAle and NetSurfP predicting the same position also not being accessible in an A*02:01, Snowflake estimates P167 being accessible (Fig 9B). In the visual representation accessibility appears plausible. Notably, positions 166 and 167 of A*01:01 are described by the Eplet 166DG. Although the corresponding amino acid positions of A*02:01 are both surface accessible, no corresponding Eplet 166EW is defined.

For position 110 of A*02:01 the predictors disagree on accessibility with PaleAle and NetSurfP negate solvent-accessibility, whilst Snowflake affirm accessibility. By visual representation, accessibility of this position however appears plausible (Fig 9C).

The positions 119 and 120 of A*02:01 appear to be masked by the beta-2-microglobulin structure (Fig 9D). Opposed to Snowflake and PaleAle both agreeing on the inaccessibility of these positions, NetSurfP predicts accessibility. Coloring the residues depending on their predicted solvent accessibility, shows the masking effect of the bound beta-2-microglobulin, rendering hidden amino acid positions, despite being potentially polymorphic, antibody-inaccessible (Fig 10).

The position-specific distributions of predicted solvent accessibility of Class I alleles at antibody-verified Eplets amino acid positions (Fig 11) show (i) Eplets with consistently high predicted solvent-accessibility (e.g. 127K, 193PV), (ii) Eplets with a consistent pattern of solvent-accessibility, where at least one amino acid position of the Eplet has high predicted solvent-accessibility (e.g. 145KHA, 44RT), and (iii) Eplets with high variance of predicted solvent accessibility at one or more positions (e.g. 144K, 163R, 65RNA).

Snowflake matching was integrated in the previously described PIRCHE Risk and Acceptable Mismatch Profile epitope matching suite. [28] The PIRCHE web service allows semi-automated HLA typing data import (GL String, HML, CSV) and provides interfaces to major HLA typing kit vendors' software suites to load HLA typing data quickly and error-free. Interactive bar charts provide interlocus and intralocus Snowflake scores along with PIRCHE scores, allele frequencies, predicted shared T cell epitopes and mean fluorescence intensities of single antigen bead assays (Fig 11).

### Discussion

A wide range of software tools characterizing potential B cell epitopes has been described in the literature (e.g. ElliPro [29], NetSurfP [11], PaleAle [12], DiscoTope [30], BepiPred [31]). Specifically for transplant histocompatibility matching, these tools supported approaches of HLA B cell epitope definition and matching. HLAMatchmaker [3] (www.epitopes.net) and EPRegistry [4,5] (www.epregistry.com.br) define a manually curated database of antibody-accessible amino acid configurations (i.e. eplets) based on a limited number of experimental HLA structures. Verifying each of these eplets by antibodies and estimating their immunogenicity however remains challenging. [7,32] The EMS-3D model considers a multidimensional score of electrostatic dissimilarity between donor and recipient HLA, which may be an additional metric to characterize the HLA surface [6]. Orthogonal to these concepts, the PIRCHE-II matching algorithm (pirche.com) predicts the number of allo-peptides being presented by self-HLA, modeling the indirect pathway of allorecognition. [28] Given CD4+ T cell help is mandatory for effective antibody development [33], this score has also been shown to correlate well with donor-specific antibody reactions in kidney transplantation. [34,35]

The pHLA3D database (phla3d.com.br) complements the experimental structures of the PDB by in silico predicted structures of pHLAs using a homology modeling pipeline approach in order to spatially locate amino acid positions in HLA proteins without available structures. [36] Snowflake however applies a different structure prediction pipeline.

The recently described HLA-EMMA (hla-emma.com) algorithm considers solvent-accessible amino acid mismatches as potential B cell receptor targets by defining solvent-accessible amino acid positions per HLA locus. An amino acid position is considered solvent-accessible, if PaleAle-or NetSurfP-solvent-accessibility scores exceed a certain threshold in at least one of the considered alleles of a locus. [10] This approach may however consider mismatched amino acids of two alleles as epitope mismatch, although the amino acids within the respective alleles aren't surface-accessible (Figs 8, 9A and 9B), as the position's accessibility was determined by a different allele. Consequently, Snowflake's allele-specific solvent-accessibility of mismatched amino-acids may increase specificity.

To incorporate simulated HLA structures into surface prediction, Snowflake implemented its own HLA surface predictor comparable to both NetSurfP 2.0 and PaleAle 4.0. These predictors implement a deep learning neural network architecture using BRNN to predict (amongst other characteristics) relative surface accessibility. Both networks have been trained with the experimental structures reported in the PDB. [11,12] Limiting on solvent accessibility as predicted output, Snowflake's deep learning model has been trained exclusively on HLA experimental structures and complemented by predicted structures. It must be considered that learning-based surface prediction is obsolete if the predicted structures have been generated for all known HLA alleles.

Although HLA Class I experimental structures showed little overall structural variance in dihedral angles between alleles (Fig 5), solvent accessibility varied across different alleles, indicating it to be allele-specific (Fig 6). Visual representations of characteristic positions support this hypothesis (Fig 9). Consequently, amino acid matching approaches may be improved by considering solvent accessibility of the respective amino acid of the specific allele, providing also a consistent explanation for epitopes' directionality.

Despite strong numeric correlation between Eplets and Snowflake, some difference in epitope definition arises. The example in Fig 9 shows strong accessibility for positions 166 (glutamic acid) and 167 (tryptophan) of A*02:01, yet there is no Eplet 166EW defined. As described by [3], the interlocus comparison with HLA-B and -C typically shows matched monomorphic configurations at these positions. However, in the meantime there are HLA-B and -C surface-expressible alleles described, which do not share the EW configuration at these positions (data not shown). Consequently, there may be scenarios, where a common hypothetical 166EW is not found in the recipient's self-Eplets, potentially provoking an immune response. Similar examples are 253Q without a corresponding common 253E or 131S without a corresponding common 131R. This indicates potential gaps in Eplet definitions, remedied by Snowflake. Furthermore, verifying Eplets distinguishably by antibody reaction patterns in cases with low frequency remains challenging. The herein presented definition of epitope mismatches provides a broadly applicable and more consistent process.

The position-specific solvent-accessibility at amino acid positions encoding for antibody-verified Eplets (Fig 11) varies for some of reported Eplets. This suggests the three-dimensional structure of such Eplets is dependent on the remaining structure of the allele they are occurring in. This raises the question, if antibody reactions against these Eplets are equally pronounced for alleles with lower accessibility compared to alleles with higher accessibility of the corresponding amino acid positions.

Our data indicates considerable structural variance between experimental structures of the same allele, which may be explained by the varying specific experimental setups on the one part and by the flexibility of HLA molecules on the other. [39,40] Though predicted solvent-accessibility varies to a greater extent across different alleles, protein flexibility and dynamics may still alter the protein surface in vivo.

Training data for surface prediction is biased towards HLA00005 (Fig 4). Excluding structures based on sequence similarity as suggested by [11,12] however would narrow the training set. Considering the training data bias being not only sequence-specific but also position-specific, as positions have individual amino acid distributions across HLA alleles, training data aggregation at the allele level is insufficient. Lacking a robust aggregation option and accepting the potential bias, the complete data sets were used for training. The applied surface area calculation considered a probe size of 1.4 Å based on the size of water molecules. Given the size of HLA-binding antibodies and the interaction area between HLA and antibody (e.g. in PDB 6ID4 [41]), it may however be considered to restrict antibody-accessibility to larger probe sizes.

The presented Snowflake prediction pipeline has been optimized for HLA Class I matching, and the approach may also apply to HLA Class II. This would preferably include replacement of structural peptide binding prediction, inclusion of HLA alpha and beta chains, large training data sets due to the heterodimeric structure and locus-specific surface prediction.

Similar to all previously defined HLA epitope matching algorithms, Snowflake also preferably processes protein-level HLA typing data. By applying the previously described haplotype frequency-based multiple imputation [42], Snowflake scores may also be calculated with low- or intermediate resolution HLA typing data. The low-resolution HLA typing data-supporting Snowflake matching algorithm for batch processing or detailed matching is available at pirche.com.

### Example 2: Snowflake epitope matching correlates with child-specific antibodies during pregnancy and donor specific antibodies after kidney transplantation.

As of today, kidney transplantation is the gold standard in renal replacement therapy. Amongst other factors, long-term graft survival is known to be dependent on patient and donor human leukocyte antigen (HLA) compatibility. HLA incompatible donor organs are recognized and damaged by the recipient's immune system - even under immunosuppressive therapy - until a loss of graft function. [1b] The definition of HLA compatibility remains challenging as not every amino acid difference between the HLA proteins of patient and donor places an equal immunologic risk. Consequently several approaches have been proposed to define histocompatibility on a functional level, each bearing its own challenges. [2b] Considering the HLA protein's surface, the HLA Matchmaker model defines epitopes as antibody-accessible, potentially discontinuous patches. [3b] The EPRegistry database (epregistry.com.br) is a publicly accessible list of these so-called Eplets. [4b,5b] Not all of these epitopes are however unambiguously confirmed in antibody responses. [6b] The recently described EMMA algorithm considers amino acid mismatches at shared surface-accessible positions as potential B cell targets. [7b] Refining that concept, the proposed Snowflake algorithm defines the HLA proteins' surface allele-specific, taking structural deviation between HLA alleles into account. [8b]

Within this Example, we evaluated the correlation between the HLA Class I-specific Snowflake scores and the frequency of developing immunizer-specific antibodies (ISA) in two independent clinical cohorts. For reference, we compared the proposed score to Eplet scores and PIRCHE-II scores. We hypothesized, (1) Snowflake scores are higher in ISA-positive patients and (2) that Snowflake scores correlate well with Eplet matching, given they both aim on defining B cell epitopes by HLA protein structure information.

### Methods

### Pregnancy cohort

The previously described pregnancy cohort (PC) of the University Hospital Basel consists of 231 pregnant women who gave live birth between September 2009 and April 2011. [9b-11b] The study was approved by the local ethics committee. Prior immunization events (blood transfusions, transplantations, or miscarriages) were ruled out. High-resolution typing for HLA-A, -B, -C, -DPA1, -DPB1, -DQA1, -DQB1, -DRB1, and -DRB3/4/5 was available for all study participants (i.e. women and children). White blood cell-derived genomic DNA was used for HLA typing by NGS. The NGSgo^{®} workflow for HLA amplification and library preparation was applied (www.gendx.com, GenDx, Utrecht, the Netherlands). The median age of the mothers was 31 years (Q1 = 28, Q3 = 35). Maternal HLA antibody specificities were assessed by single antigen beads (LabScreenTM Single HLA Antigen Beads (SAB), OneLambda Thermo Fisher, Canoga Park, CA, USA)) between days 1 and 4 after delivery and mapped to the child HLA typing to identify child-specific antibodies (CSA). Previous reports on this cohort have shown a correlation between predictions of different epitope matching algorithms and CSA [9b-11b].

### Kidney transplant cohort

The kidney transplant cohort (KTC) of the Charité University Hospital consists of 305 patients who underwent kidney transplantation between 2000 and 2019. The study was approved by the institutional review boards of the Charité hospital. Patients were regularly screened for HLA antibodies prior to transplantation and during their follow-up (Luminex^{®}-based LABScreenTM mixed and SAB assay, OneLambda Thermo Fisher, Canoga Park, CA). HLA antibody specificities were mapped to kidney donor HLA typings to identify donor-specific antibodies (DSA). Enrolled patients did not have DSA prior to transplantation and had a negative complement-dependent-cytotoxicity crossmatch using isolated T and B lymphocytes. HLA typing of the recipient was obtained by serological (HLA Class I) and DNA-based techniques (HLA Classes I and II). Sequence-specific primer (SSP) (Olerup, Stockholm, Sweden) and reverse sequence-specific oligonucleotide (SSO) (One Lambda Thermo Fisher, Canoga Park, CA) assays were used according to the manufacturer's instructions. Recipients and donors were typed twice. HLA typing of deceased donors was provided by the donor center and confirmed by SSO. Living donors were typed by serology and SSO or SSP.

To consider all applied donor allocation schemas equally, the cohort comprises of four matched groups: (1) highly sensitized patients that have been transplanted via the Acceptable Mismatch allocation program of Eurotransplant (94 patients), (2) Eurotransplant Kidney Allocation System (ETKAS)-allocated patients transplanted with a reported panel-reactive antibody level (PRA) of ≤ 5 %(92 patients), (3) ETKAS-allocated patients transplanted with a reported PRA of >5 % and < 85 % (87 patients), and (4) highly-sensitized patients with PRA levels ≥ 85 % allocated through ETKAS (32 patients). Additional demographic data on the KTC is provided in table 1. In the full kidney transplant cohort of the Charité University Hospital, both B cell and T cell epitope matching algorithms were previously shown to correlate with DSA development and graft survival [12b].

**Table 1: KTC characteristics and demographics. Antigen mismatches considering serologic split typing. SD, standard deviation; IQR, interquartile range.**

| **Characteristic** | **Value** |
|---|---|
| follow up, mean years, (SD) | 6.6 (4.5) |
| recipient age at TX, mean years, (SD) | 47.3 (12.2) |
| recipient male, number, (percentage) | 164 (53.8 %) |
| primary graft function, number, (percentage) | 140 (45.9 %) |
| cold ischemia time, hrs, (SD) | 14.7 (4.8) |
| donor age at TX, mean years, (SD) | 46.8 (13.5) |
| donor male, number, (percentage) | 160 (52.5 %) |
| waiting time, mean months, (SD) | 56.3 (39.3) |
| antigen mismatches A-B, median, (IQR) | 1 (1) |
| antigen mismatches A-B-DR, median, (IQR) | 2 (1) |
| PRA at TX, median percentage, (IQR) | 27 % (41 %) |
| peak PRA, median percentage, (IQR) | 70 % (48.5 %) |
| blood group identical, number, (percentage) | 45 (85.2 %) |

### Snowflake epitope matching algorithm

As described above, the Snowflake algorithm is an integrated prediction pipeline counting the number of mismatched solvent accessible amino acids between donor and recipient. For that purpose, a neural network predictor was trained on experimental HLA structures of the Protein Data Bank (www.rcsb.org) supplemented by HLA structures predicted by the AlphaFold predictor. [13b-15b] Solvent accessibility was evaluated allele-specific, resulting in HLA-individual amino acid positions to be matched. Comparison with recipient HLA considered HLA-A, -B and -C simultaneously (i.e. interlocus).

### Reference epitope matching algorithms

For comparison of the Snowflake algorithm with other epitope matching concepts within the clinical validation cohorts, the number of interlocus-mismatched antibody-verified donor eplets (EPS) as defined by the HLA Epitope Registry (www.epregistry.com.br, version 3.0) was determined [5b] and the number of PIRCHE-II (www.pirche.com, version 3.46) was calculated (reviewed in [16b]). In the PC, high resolution typing data on 11 loci allowed for PIRCHE analysis of DRB1 (note PIRCHE-II is identical to PIRCHE-DRB1), DRB3/4/5, DQ and DP as independent donor-derived peptide presenters, as suggested by [11b], yielding four separate PIRCHE matching scores.

### Imputation of HLA typing data in the KTC

The underlying core algorithms of all epitope matching methods require protein-level HLA typing. To apply epitope matching despite having only intermediate resolution level typings in the KTC, the previously described multiple imputation approach was refined. [17b] Multi allele codes were converted into antigen-specific candidate lists (https://hml.nmdp.org/MacUI/, IPD-IMGT/HLA version 3.46). [18b] Potential haplotype pairs matching the low-resolution-converted input typings were filtered by candidate lists respectively. The remaining haplotype pairs' frequencies were normalized by all remaining haplotype pairs. High-resolution pairs were only considered if the normalized frequency exceeds 1%. Epitope matching scores were calculated for each high-resolution recipient-donor-combination and summed up weighted by respective pair's frequency.

### Pooling ISA data

Snowflake score distributions in ISA-negative and ISA-positive cases were visualized by boxplots for each HLA locus individually. Regression analyses were carried out on pooled datasets of the respective cohort to increase sample size. To that extent, each mismatch of the PC and its corresponding CSA status was considered individually. Given the PC is haplotype-matched by design, each pregnancy translates into up to three data points in the pooled PC dataset, excluding homozygous and matched data points. Prior to aggregation, Snowflake and EPS were centered by subtracting the respective mean and scaled by dividing the respective standard deviation to account for locus-specific ranges. PIRCHE scores were log-transformed based on previously reported logarithmic hazards. [12b] For the KTC, the respective locus' epitope scores were normalized similarly. As organ donors may be mismatched on both HLA per locus, epitope scores were defined locus-specific rather than mismatch-specific. Corresponding, the DSA status considers DSA against only one or both HLA of the donor as positive. Given low-resolution HLA typing data in the KTC, defining HLA mismatches is non-trivial, as allele-specific mismatches may still occur in zero mismatch donor-recipient pairs. To still exclude the bias of HLA-matched data points, cases with zero PIRCHE, zero EPS and zero Snowflake were excluded from analyses.

### Statistical analysis

In the pooled PC dataset, stepwise binomial logistic regression based on Akaike information criterion was applied to evaluate independence of Snowflake and reference epitope matching algorithms. In the KTC dataset regression analyses were carried out by Cox proportional hazard and Cox multiple regression. Wilcoxon signed-rank test was applied to differences in epitope match score distributions between ISA negative and ISA positive cases. Correlation between epitope matching scores was analyzed by Spearman's rank-correlation coefficient (Spearman's Rho rs). p-Values of less than 0.05 were considered statistically significant. Statistical calculations for the PC were executed in R software (R 3.6.1, R Foundation for Statistical Computing, Vienna, Austria), for the KTC in SPSS software (SPSS 27.0.0.0, IBM Corp., Armonk, NY).

### Results

Snowflake score calculations considered the median solvent accessibility score (0.3717) in the panel of reference alleles as reported by [8b]. The interlocus-restricted locus-specific Snowflake scores in the PC were significantly higher in the ISA-positive compared to the ISA-negative groups for HLA-A, -B and -C (Fig 17), considering only locus-mismatched cases.

Within the pooled PC dataset, 566 HLA-mismatches were analyzed within a single model, corresponding to 121 child-specific antibodies. Correlation analysis revealed weak to moderate correlation between Snowflake and PIRCHE (0.36 <= rs <= 0.49, p < 0.001) and a strong correlation with EPS (rs = 0.72, p < 0.001) (Fig 19). Univariable logistic regression identified all considered models (except for PIRCHE-DRB3/4/5) as statistically significant.

**Table 2: Univariable binomial logistic regression and minimal model created by stepwise binomial logistic regression of considered epitope matching algorithm scores and the development of child-specific HLA antibodies. CI: 95% confidence interval**

| | Univariable regression | | | Multiple logistic regression | | |
|---|---|---|---|---|---|---|
| Algorithm | Odds ratio | CI | Significance (P) | Odds ratio | CI | Significanc e (p) |
| PIRCHE-DRB1 | 1.40 | 1.06-1.88 | 0.020 | | | |
| PIRCHE-DRB3/4/5 | 1.24 | 0.98-1.58 | 0.075 | | | |
| PIRCHE-DQ | 1.35 | 1.11-1.67 | 0.004 | | | |
| PIRCHE-DP | 1.46 | 1.15-1.86 | 0.002 | 1.25 | 0.98-1.62 | 0.079 |
| EPS | 1.59 | 1.29-1.96 | < 0.001 | | | |
| Snowflake | 1.63 | 1.33-1.99 | < 0.001 | 1.55 | 1.25-1.91 | < 0.001 |

Also in the KTC, statistically significantly higher HLA-A- and HLA-C-specific Snowflake scores were observed in cases with HLA-A- and HLA-C-specific DSA, respectively. Despite higher median HLA-B-specific Snowflake scores in HLA-B-specific DSA positive cases, the difference of distributions was not statistically significant (Fig 18).

In the pooled KTC dataset, 622 locus-specific epitope match scores were correlated with 66 DSA events. Correlation analysis indicated a strong correlation between PIRCHE presented by HLA-DRB1, EPS and Snowflake (Table 5), with highest rs between EPS and Snowflake (rs = 0.806). In Cox regression, all three scores were significantly correlated with dnDSA (Table 4). Cox multiple regression identified PIRCHE and EPS as contributors to a joint model, whereas Snowflake did not significantly contribute to the model performance (Table 4). A strong dependence of Snowflake and Eplets is also observed by pairwise Cox multiple regression (Table 3).

**Table 3: Pairwise Cox multiple regression of (1) PIRCHE-DRB1 and EPS, (2) PIRCHE-DRB1 and Snowflake, and (3) EPS and Snowflake predicting dnDSA. Whilst PIRCHE-DRB1 and EPS seem to contribute individually, EPS and Snowflake appear to be highly correlated. CI, 95% confidence interval.**

| Pair | Algorithm | Pairwise Cox multiple regression | | |
|---|---|---|---|---|
| | | Odds ratio | CI | Significance (p) |
| 1 | PIRCHE-DRB1 | 1.34 | 0.96-1.88 | 0.090 |
| | EPS | 1.34 | 1.01-1.78 | 0.045 |
| 2 | PIRCHE-DRB1 | 1.51 | 1.09-2.08 | 0.012 |
| | Snowflake | 1.11 | 0.88-1.41 | 0.386 |
| 3 | EPS | 1.54 | 1.13-2.10 | 0.007 |
| | Snowflake | 1.01 | 0.76-1.35 | 0.938 |

**Table 4: Cox regression and Cox multiple regression analyses of PIRCHE-DRB1, EPS and Snowflake predicting donor-specific antibodies in the KTC. Despite all three scores individually being significantly correlated with DSA, in a combined model Snowflake does not contribute significantly. CI, 95% confidence interval.**

| | Cox regression | | | Cox multiple regression | | |
|---|---|---|---|---|---|---|
| Algorithm | Odds ratio | CI | Significance (P) | Odds ratio | CI | Significanc e (p) |
| PIRCHE-DRB1 | 1.61 | 1.21-2.15 | 0.001 | 1.35 | 0.96-1.90 | 0.087 |
| EPS | 1.51 | 1.23-1.95 | < 0.001 | 1.37 | 0.98-1.93 | 0.068 |
| Snowflake | 1.30 | 1.07-1.58 | 0.007 | 0.96 | 0.71-1.30 | 0.800 |

**Table 5: Correlation matrix of scaled (EPS, SNF, PIRCHE-DRB1) and logarithmic (PIRCHE-DRB1) epitope match scores in the KTC. Numbers indicate Spearman's rho (rs). **: p < 0.01.**

| Spearman's rho | PIRCHE-DRB1 | EPS |
|---|---|---|
| EPS | 0.620** | - |
| Snowflake | 0.618** | 0.806** |

### Discussion

Confirming our hypothesis, Snowflake scores were significantly higher in mismatched cases of both cohorts across HLA-A, -B and -C if they developed ISA against the respective locus (Figs 17 and 18, except for HLA-B in the KTC). Consequently, there is added value in prediction of ISA to classical HLA antigen matching by applying Snowflake matching.

Correlation analyses revealed a moderate to strong correlation between all evaluated epitope matching approaches. Notably, in both cohorts Spearman's rho was highest between Snowflake and Eplets. Both approaches aim on predicting HLA protein surface patches that are antibody accessible and thus potential targets for B cell receptors. Univariable regression analyses confirmed that all tested epitope matching strategies are significantly predictive of ISA (Tables 2 and 3). Multiple regression however is at present inconclusive about the independence of tested predictors. While in the PC the combination of Snowflake and PIRCHE-DP were a model with good CSA prediction (Table 2), in the KTC Eplets were preferably combined with PIRCHE-DRB1 to predict DSA (Table 3). Previous studies found an independent impact of Eplets and PIRCHE for the prediction of DSA in organ transplantation [12b,19b,20b], and Snowflake appears to provide a similar effect. Restricting the Snowflake algorithm to intralocus comparison also resulted in significant correlation with ISA with reduced correlation to the reference epitope matching algorithms.

Similar to the recently described HLA EMMA matching algorithm [7b], Snowflake predicts the number of solvent-accessible amino acid mismatches between donor and recipient. Opposed to EMMA however, each HLA's surface is evaluated individually, rather than per locus. As indicated by [8b], the individual amino acid configuration may alter the solvent-accessibility profile of HLA alleles. Although the presence of DSA is known to be a major risk factor for graft rejection and loss of graft function [24b-27b] in all organ transplant domains, it must be noted the present analyses did not yet cover the clinical impact of each of the developed DSA.

In summary, we present the first clinical evidence of the Snowflake score correlating with ISA development. The strength of this study is the parallel analysis of two independent cohorts of pregnant women and patients who received kidney transplantation. In both cohorts, the number of allele-specific surface-accessible amino acid mismatches (i.e. Snowflake score) was significantly associated with ISA development

### References for Example 1:

1. Süsal C, Opelz G. Current role of human leukocyte antigen matching in kidney transplantation. Current Opinion in Organ Transplantation. 2013 Aug;18(4):438-44.
2. Tambur AR, Claas FHJ. HLA Epitopes as Viewed by Antibodies: What Is it All About?: HLA Epitopes as Viewed by Antibodies. American Journal of Transplantation. 2015 May;15(5):1148-54.
3. Duquesnoy RJ. HLAMatchmaker: a molecularly based algorithm for histocompatibility determination. I. Description of the algorithm. Human Immunology. 2002 May;63(5):339-52.
4. Duquesnoy RJ. Update of the HLA class I eplet database in the website based registry of antibody-defined HLA epitopes: HLA class I eplet database update. Tissue Antigens. 2014 Jun;83(6):382-90.
5. Duquesnoy RJ, Marrari M, Marroquim MS, Borges AG, da Mata Sousa LCD, Socorro A, et al. Second update of the International Registry of HLA Epitopes. I. The HLA-ABC Epitope Database. Human Immunology. 2019 Feb;80(2):103-6.
6. Mallon DH, Kling C, Robb M, Ellinghaus E, Bradley JA, Taylor CJ, et al. Predicting Humoral Alloimmunity from Differences in Donor and Recipient HLA Surface Electrostatic Potential. JI. 2018 Dec 15;201(12):3780-92.
7. Bezstarosti S, Bakker KH, Kramer CSM, de Fijter JW, Reinders MEJ, Mulder A, et al. A Comprehensive Evaluation of the Antibody-Verified Status of Eplets Listed in the HLA Epitope Registry. Front Immunol. 2022 Jan 28;12:800946.
8. Tan J, Qiu J, Tang X. HLA Amino Acid Residue Matching in 2575 Kidney Transplants. Transplantation Proceedings. 2007 Jun;39(5):1429-31.
9. Kosmoliaptsis V, Sharples LD, Chaudhry A, Johnson RJ, Fuggle SV, Halsall DJ, et al. HLA class I amino acid sequence-based matching after interlocus subtraction and long-term outcome after deceased donor kidney transplantation. Human Immunology. 2010 Sep;71(9):851-6.
10. Kramer CSM, Koster J, Haasnoot GW, Roelen DL, Claas FHJ, Heidt S. HLA-EMMA : A user-friendly tool to analyse HLA class I and class II compatibility on the amino acid level. HLA. 2020 Jul;96(1):43-51.
11. Klausen MS, Jespersen MC, Nielsen H, Jensen KK, Jurtz VI, Sønderby CK, et al. NetSurfP-2.0: Improved prediction of protein structural features by integrated deep learning. Proteins. 2019 Jun;87(6):520-7.
12. Mirabello C, Pollastri G. Porter, PaleAle 4.0: high-accuracy prediction of protein secondary structure and relative solvent accessibility. Bioinformatics. 2013, 15;29(16):2056-8.
13. Jumper J, Evans R, Pritzel A, Green T, Figurnov M, Ronneberger O, et al. Highly accurate protein structure prediction with AlphaFold. Nature. 2021 Aug 26;596(7873):583-9.
14. Varadi M, Anyango S, Deshpande M, Nair S, Natassia C, Yordanova G, et al. AlphaFold Protein Structure Database: massively expanding the structural coverage of protein-sequence space with high-accuracy models. Nucleic Acids Research. 2021 Nov 17;gkab1061.
15. Evans R, O'Neill M, Pritzel A, Antropova N, Senior A, Green T, et al. Protein complex prediction with AlphaFold-Multimer [Internet]. Bioinformatics; 2021 Oct [cited 2021 Dec 20]. Available from: http://biorxiv.org/lookup/doi/10.1101/2021.10.04.463034
16. Berman HM. The Protein Data Bank. Nucleic Acids Research. 2000 Jan 1;28(1):235-42.
17. Robinson J, Barker DJ, Georgiou X, Cooper MA, Flicek P, Marsh SGE. IPD-IMGT/HLA Database. Nucleic Acids Research. 2019 Oct 31;gkz950.
18. Camacho C, Coulouris G, Avagyan V, Ma N, Papadopoulos J, Bealer K, et al. BLAST+: architecture and applications. BMC Bioinformatics. 2009 Dec;10(1):421.
19. Cock PJA, Antao T, Chang JT, Chapman BA, Cox CJ, Dalke A, et al. Biopython: freely available Python tools for computational molecular biology and bioinformatics. Bioinformatics. 2009 Jun 1;25(11):1422-3.
20. Golub GH, Van Loan CF. Matrix computations. Fourth edition. Baltimore: The Johns Hopkins University Press; 2013. 756 p. (Johns Hopkins studies in the mathematical sciences).
21. Vita R, Mahajan S, Overton JA, Dhanda SK, Martini S, Cantrell JR, et al. The Immune Epitope Database (IEDB): 2018 update. Nucleic Acids Res. 2019 Jan 8;47(D1):D339-43.
22. Abella J, Antunes D, Clementi C, Kavraki L. APE-Gen: A Fast Method for Generating Ensembles of Bound Peptide-MHC Conformations. Molecules. 2019 Mar 2;24(5):881.
23. Kufareva I, Abagyan R. Methods of Protein Structure Comparison. In: Orry AJW, Abagyan R, editors. Homology Modeling [Internet]. Totowa, NJ: Humana Press; 2011 [cited 2022 Jan 10]. p. 231-57. (Methods in Molecular Biology; vol. 857). Available from: http://link.springer.com/10.1007/978-1-61779-588-6_10
24. Shrake A, Rupley JA. Environment and exposure to solvent of protein atoms. Lysozyme and insulin. Journal of Molecular Biology. 1973 Sep;79(2):351-71.
25. Kunzmann P, Hamacher K. Biotite: a unifying open source computational biology framework in Python. BMC Bioinformatics. 2018 Dec;19(1):346.
26. Schuster M, Paliwal KK. Bidirectional recurrent neural networks. IEEE Trans Signal Process. 1997 Nov;45(11):2673-81.
27. Matern BM, Mack SJ, Osoegawa K, Maiers M, Niemann M, Robinson J, et al. Standard reference sequences for submission of HLA genotyping for the 18th International HLA and Immunogenetics Workshop. HLA. 2021 Jun;97(6):512-9.
28. Geneugelijk K, Spierings E. Matching donor and recipient based on predicted indirectly recognizable human leucocyte antigen epitopes. Int J Immunogenet. 2018 Apr;45(2):41-53.
29. Ponomarenko J, Bui H-H, Li W, Fusseder N, Bourne PE, Sette A, et al. ElliPro: a new structure-based tool for the prediction of antibody epitopes. BMC Bioinformatics. 2008 Dec;9(1):514.
30. Kringelum JV, Lundegaard C, Lund O, Nielsen M. Reliable B Cell Epitope Predictions: Impacts of Method Development and Improved Benchmarking. Peters B, editor. PLoS Comput Biol. 2012 Dec 27;8(12):e1002829.
31. Jespersen MC, Peters B, Nielsen M, Marcatili P. BepiPred-2.0: improving sequence-based B-cell epitope prediction using conformational epitopes. Nucleic Acids Research. 2017 Jul 3;45(W1):W24-9.
32. Mohammadhassanzadeh H, Oualkacha K, Zhang W, Klement W, Bourdiec A, Lamsatfi J, et al. On Path to Informing Hierarchy of Eplet Mismatches as Determinants of Kidney Transplant Loss. Kidney International Reports. 2021 Jun;6(6):1567-79.
33. Chen C-C, Koenig A, Saison C, Dahdal S, Rigault G, Barba T, et al. CD4+ T Cell Help Is Mandatory for Naive and Memory Donor-Specific Antibody Responses: Impact of Therapeutic Immunosuppression. Front Immunol. 2018 Feb 19;9:275.
34. Otten HG, Calis JJA, Ke 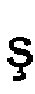 mir C, van Zuilen AD, Spierings E. Predicted indirectly recognizable HLA epitopes presented by HLA-DR correlate with the de novo development of donor-specific HLA IgG antibodies after kidney transplantation. Human Immunology. 2013 Mar;74(3):290-6.
35. Lachmann N, Niemann M, Reinke P, Budde K, Schmidt D, Halleck F, et al. Donor-Recipient Matching Based on Predicted Indirectly Recognizable HLA Epitopes Independently Predicts the Incidence of De Novo Donor-Specific HLA Antibodies Following Renal Transplantation. Am J Transplant. 2017 Dec;17(12):3076-86.
36. Menezes Teles e Oliveira D, Melo Santos de Serpa Brandão R, Claudio Demes da Mata Sousa L, das Chagas Alves Lima F, Jamil Hadad do Monte S, Sérgio Coelho Marroquim M, et al. pHLA3D: An online database of predicted three-dimensional structures of HLA molecules. Human Immunology. 2019 Oct;80(10):834-41.
37. Alexander LT, Lepore R, Kryshtafovych A, Adamopoulos A, Alahuhta M, Arvin AM, et al. Target highlights in CASP14 : Analysis of models by structure providers. Proteins. 2021 Dec;89(12):1647-72.
38. Unger R, Moult J. Finding the lowest free energy conformation of a protein is an NP-hard problem: Proof and implications. Bltn Mathcal Biology. 1993 Nov;55(6):1183-98.
39. Wieczorek M, Abualrous ET, Sticht J, Alvaro-Benito M, Stolzenberg S, Noé F, et al. Major Histocompatibility Complex (MHC) Class I and MHC Class II Proteins: Conformational Plasticity in Antigen Presentation. Front Immunol [Internet]. 2017 Mar 17 [cited 2022 Feb 14];8. Available from: http://journal.frontiersin.org/article/10.3389/fimmu.2017.00292/full
40. Natarajan K, Jiang J, May NA, Mage MG, Boyd LF, McShan AC, et al. The Role of Molecular Flexibility in Antigen Presentation and T Cell Receptor-Mediated Signaling. Front Immunol. 2018 Jul 17;9:1657.
41. Gu Y, Wong YH, Liew CW, Chan CEZ, Murali TM, Yap J, et al. Defining the structural basis for human alloantibody binding to human leukocyte antigen allele HLA-A*11:01. Nat Commun. 2019 Dec;10(1):893.
42. Geneugelijk K, Wissing J, Koppenaal D, Niemann M, Spierings E. Computational Approaches to Facilitate Epitope-Based HLA Matching in Solid Organ Transplantation. Journal of Immunology Research. 2017;2017:1-9.

### References for Example 2:

1b. Süsal C, Opelz G. Current role of human leukocyte antigen matching in kidney transplantation. Current Opinion in Organ Transplantation. 2013 Aug;18(4):438-44.
2b. Tambur AR. HLA-Epitope Matching or Eplet Risk Stratification: The Devil Is in the Details. Front Immunol. 2018 Aug 31;9:2010.
3b. Duquesnoy RJ. HLAMatchmaker: a molecularly based algorithm for histocompatibility determination. I. Description of the algorithm. Human Immunology. 2002 May;63(5):339-52.
4b. Duquesnoy RJ. Update of the HLA class I eplet database in the website based registry of antibody-defined HLA epitopes: HLA class I eplet database update. Tissue Antigens. 2014 Jun;83(6):382-90.
5b. Duquesnoy RJ, Marrari M, Marroquim MS, Borges AG, da Mata Sousa LCD, Socorro A, et al. Second update of the International Registry of HLA Epitopes. I. The HLA-ABC Epitope Database. Human Immunology. 2019 Feb;80(2):103-6.
6b. Bezstarosti S, Bakker KH, Kramer CSM, de Fijter JW, Reinders MEJ, Mulder A, et al. A Comprehensive Evaluation of the Antibody-Verified Status of Eplets Listed in the HLA Epitope Registry. Front Immunol. 2022 Jan 28;12:800946.
7b. Kramer CSM, Koster J, Haasnoot GW, Roelen DL, Claas FHJ, Heidt S. HLA-EMMA : A user-friendly tool to analyse HLA class I and class II compatibility on the amino acid level. HLA. 2020 Jul;96(1):43-51.
8b. Niemann M. Snowflake: A deep learning-based B cell epitope matching algorithm considering allele-specific surface accessibility. manuscript in preparation. 2022;
9b. Geneugelijk K, Hönger G, van Deutekom HWM, Thus KA, Kemir C, Hösli I, et al. Predicted Indirectly Recognizable HLA Epitopes Presented by HLA-DRB1 Are Related to HLA Antibody Formation During Pregnancy: PIRCHE-II in HLA Antibody Formation. American Journal of Transplantation. 2015 Dec;15(12):3112-22.
10b. Hönger G, Niemann M, Schawalder L, Jones J, Heck MR, Pasch LAL, et al. Toward defining the immunogenicity of HLA epitopes: Impact of HLA class I eplets on antibody formation during pregnancy. HLA. 2020 Nov;96(5):589-600.
11b. Niemann M, Matern BM, Spierings E, Schaub S, Hönger G. Peptides Derived From Mismatched Paternal Human Leukocyte Antigen Predicted to Be Presented by HLA-DRB1, -DRB3/4/5, -DQ, and -DP Induce Child-Specific Antibodies in Pregnant Women. Front Immunol. 2021 Dec 21;12:797360.
12b. Lachmann N, Niemann M, Reinke P, Budde K, Schmidt D, Halleck F, et al. Donor-Recipient Matching Based on Predicted Indirectly Recognizable HLA Epitopes Independently Predicts the Incidence of De Novo Donor-Specific HLA Antibodies Following Renal Transplantation. Am J Transplant. 2017 Dec;17(12):3076-86.
13b. Evans R, O'Neill M, Pritzel A, Antropova N, Senior A, Green T, et al. Protein complex prediction with AlphaFold-Multimer [Internet]. Bioinformatics; 2021 Oct [cited 2021 Dec 20]. Available from: http://biorxiv.org/lookup/doi/10.1101/2021.10.04.463034
14b. Jumper J, Evans R, Pritzel A, Green T, Figurnov M, Ronneberger O, et al. Highly accurate protein structure prediction with AlphaFold. Nature. 2021 Aug 26;596(7873):583-9.
15b. Varadi M, Anyango S, Deshpande M, Nair S, Natassia C, Yordanova G, et al. AlphaFold Protein Structure Database: massively expanding the structural coverage of protein-sequence space with high-accuracy models. Nucleic Acids Research. 2021 Nov 17;gkab1061.
16b. Geneugelijk K, Spierings E. Matching donor and recipient based on predicted indirectly recognizable human leucocyte antigen epitopes. Int J Immunogenet. 2018 Apr;45(2):41-53.
17b. Geneugelijk K, Wissing J, Koppenaal D, Niemann M, Spierings E. Computational Approaches to Facilitate Epitope-Based HLA Matching in Solid Organ Transplantation. Journal of Immunology Research. 2017;2017:1-9.
18b. Bochtler W, Maiers M, Bakker JNA, Baier DM, Hofmann JA, Pingel J, et al. An update to the HLA Nomenclature Guidelines of the World Marrow Donor Association, 2012. Bone Marrow Transplant. 2013 Nov;48(11):1387-8.
19b. Sakamoto S, Iwasaki K, Tomosugi T, Niemann M, Spierings E, Miwa Y, et al. Analysis of T and B Cell Epitopes to Predict the Risk of de novo Donor-Specific Antibody (DSA) Production After Kidney Transplantation: A Two-Center Retrospective Cohort Study. Front Immunol. 2020 Aug 27;11:2000.
20b. Mangiola M. Pittsburgh CTOC Study. 2022;in review.
21b. Geneugelijk K, Niemann M, Drylewicz J, van Zuilen AD, Joosten I, Allebes WA, et al. PIRCHE-II Is Related to Graft Failure after Kidney Transplantation. Front Immunol. 2018 Mar 5;9:321.
22b. Prommool S, Jhangri GS, Cockfield SM, Halloran PF. Time Dependency of Factors Affecting Renal Allograft Survival. JASN. 2000 Mar;11(3):565-73.
23b. Legendre C, Canaud G, Martinez F. Factors influencing long-term outcome after kidney transplantation. Transpl Int. 2014 Jan;27(1):19-27.
24b. Lachmann N, Terasaki PI, Budde K, Liefeldt L, Kahl A, Reinke P, et al. AntiHuman Leukocyte Antigen and Donor-Specific Antibodies Detected by Luminex Posttransplant Serve as Biomarkers for Chronic Rejection of Renal Allografts. Transplantation. 2009 May 27;87(10):1505-13.
25b. Zhang R. Donor-Specific Antibodies in Kidney Transplant Recipients. CJASN. 2018 Jan 6;13(1):182-92.
26b. O'Leary JG, Klintmalm GB. Impact of donor-specific antibodies on results of liver transplantation. Current Opinion in Organ Transplantation. 2013 Jun;18(3):279-84.
27b. Barten MJ, Schulz U, Beiras-Fernandez A, Berchtold-Herz M, Boeken U, Garbade J, et al. The clinical impact of donor-specific antibodies in heart transplantation. Transplantation Reviews. 2018 Oct;32(4):207-17.
28b. Schawalder L, Hönger G, Kleiser M, Heck MR, Pasch LAL, Vendelbosch S, et al. Development of an immunogenicity score for HLA-DQ eplets: A conceptual study. HLA. 2021 Jan;97(1):30-43.
29b. Lemieux W, Mohammadhassanzadeh H, Klement W, Daniel C, Sapir-Pichhadze R. Matchmaker, matchmaker make me a match: Opportunities and challenges in optimizing compatibility of HLA eplets in transplantation. Int J Immunogenet. 2021 Apr;48(2):135-44.
30b. Bezstarosti S, Kramer CSM, Claas FHJ, de Fijter JW, Reinders MEJ, Heidt S. Implementation of molecular matching in transplantation requires further characterization of both immunogenicity and antigenicity of individual HLA epitopes. Human Immunology. 2021 Dec;S0198885921002925.
31b. Mohammadhassanzadeh H, Oualkacha K, Zhang W, Klement W, Bourdiec A, Lamsatfi J, et al. On Path to Informing Hierarchy of Eplet Mismatches as Determinants of Kidney Transplant Loss. Kidney International Reports. 2021 Jun;6(6):1567-79.
32b. Niemann M, Lachmann N, Geneugelijk K, Spierings E. Computational Eurotransplant kidney allocation simulations demonstrate the feasibility and benefit of T-cell epitope matching. Merks RMH, editor. PLoS Comput Biol. 2021 Jul 27;17(7):e1009248.
33b. Tran JN, Günther OP, Sherwood KR, Fenninger F, Allan LL, Lan J, et al. High-throughput sequencing defines donor and recipient HLA B-cell epitope frequencies for prospective matching in transplantation. Commun Biol. 2021 Dec;4(1):583.

## Claims

1. A computer-implemented method for producing a database of predicted solvent accessible surface residues for multiple human leukocyte antigen (HLA) proteins, comprising
a. Providing one or more HLA protein structures, for which a structure has been experimentally determined,
b. Predicting HLA protein structure of one or more HLA proteins, for which a structure has not been experimentally determined,
c. Supplementing the predicted HLA protein structures of b. with an HLA-bound peptide, if the structure of b. does not exhibit an HLA-bound peptide,
d. Determining solvent accessible surface residues of each HLA protein structure of a. and c., and
e. Generating a database of solvent accessible surface residues for the HLA proteins of a. and c. and HLA proteins additional to those of a. to c., comprising employing a neural network trained on amino acids sequences of the HLA proteins of a. and c. and the corresponding solvent accessible surface residues determined in d.

2. The method according to claim 1, wherein the HLA protein structure of a. to c. and the solvent accessible surface residues of d. comprise (i) an extracellular alpha chain, a beta-2-microglobulin and an HLA-bound peptide structure, in case of HLA Class I proteins, or (ii) the protein's extracellular alpha and beta chains, and an HLA-bound peptide structure, in case of HLA Class II proteins.

3. The method according to any of the preceding claims, wherein the supplementing according to c. comprises:
a. For predicted structures with known binding peptides, adding said known peptides to the HLA protein structure, and/or
b. For predicted structures without known binding peptides, adding peptides to the HLA protein structure that are known to bind to an HLA allele with a high degree (the greatest degree) of amino acid sequence identity in extracellular domains to the HLA amino acid sequence of said predicted structure without known binding peptides.

4. The method according to any of the preceding claims, wherein determining solvent accessible surface residues of an HLA protein structure according to d. comprises employing a rolling-probe algorithm, preferably a Shrake-Rupley rolling-probe algorithm, a Half Sphere Exposure (HSE) algorithm, a linear approximation algorithm or a power diagram-based algorithm.

5. The method according to any of the preceding claims, wherein the neural network according to e. comprises a long short-term memory bidirectional recurrent neural network.

6. The method according to any of the preceding claims, wherein the neural network according to e. comprises the HLA alpha chain (for HLA Class I) or the HLA alpha and beta chains (for HLA Class II) amino acid sequence(s) as an input and the solvent accessible surface residues as an output.

7. The method according to any of the preceding claims, wherein the database according to e. comprises solvent accessible surface residues for essentially all HLA proteins (all HLA alleles) in any given HLA Class.

8. The method according to any of the preceding claims, wherein the neural network according to e. is HLA Class and/or HLA-locus specific, for example comprises multiple solvent accessible surface residues for multiple human leukocyte antigen (HLA) proteins from only HLA Class I proteins, only HLA Class II proteins, or only for alleles selected from the same HLA locus.

9. The method according to any of the preceding claims, wherein the method is automated, preferably wherein the method does not comprise manual curation of the database according to verified antibody binding.

10. A computer-implemented method for predicting a human leukocyte antigen (HLA) B-cell epitope that is mismatched between two or more subjects, comprising
i. Determining one or more mismatched amino acids of an HLA protein between two or more subjects,
- comprising preferably identifying amino acid similarity of an HLA protein of one subject with the same amino acid positions in one or more HLA proteins of another subject,
ii. Determining whether said mismatched amino acids are predicted to be positioned on a solvent accessible surface of an HLA structure,
- comprising comparing said mismatched amino acids to a database of solvent accessible surface residues for HLA proteins, wherein the solvent accessible surface residues of the database are predicted by a neural network trained on amino acids sequences of experimentally determined and predicted HLA protein structures, and on the corresponding residues' surface accessibility,
iii. wherein a mismatched amino acid predicted to be surface accessible indicates a human leukocyte antigen (HLA) B-cell epitope that is mismatched between two or more subjects.

11. The method according to claim 10, wherein the database is produced using a method according to any of claims 1 to 9.

12. The method according to claim 10 or 11, wherein a mismatched amino acid predicted to be surface accessible indicates an antibody immune response against the corresponding mismatched HLA protein.

13. A computer-implemented method for selecting transplantation material for allogeneic transplantation between subjects with one or more human leukocyte antigen (HLA) mismatches, comprising assessing whether one or more human leukocyte antigen (HLA) B-cell epitopes are mismatched between said subjects according to the method of claims 10-12, wherein the number of determined mismatched HLA B-cell epitopes positively correlates with an antibody immune response against a corresponding mismatched HLA protein and/or a likelihood of an adverse immune reaction after transplantation.

14. Method according to claim 13, wherein the transplantation comprises solid organ transplantation (such as kidney transplantation, liver transplantation, heart transplantation, lung transplantation), transplantation of stem cells (such as transplantation of hematopoietic stem-cells (HSCT), cord blood or cord blood cells), or transfusion of blood or cell products (such as platelet transfusion).

15. The method according to claims 13 or 14, comprising additionally determining one or more mismatched HLA T-cell epitopes between said subjects, preferably comprising determining the number of predicted indirectly recognized HLA epitopes (PIRCHEs), wherein said PIRCHEs are recipient- or donor-specific HLA-derived peptides from a mismatched recipient or donor HLA allele, respectively, and are predicted to be presented by an HLA molecule, wherein the number of determined mismatched HLA T-cell epitopes positively correlates with a likelihood of an adverse immune reaction after transplantation.
